(19) Europäisches Patentamt
European Patent Office
Office européen des brevets

(11) **EP 1 413 629 A1**

(12) **EUROPEAN PATENT APPLICATION**
published in accordance with Art. 158(3) EPC

(43) Date of publication:
**28.04.2004 Bulletin 2004/18**

(21) Application number: **02755778.4**

(22) Date of filing: **02.08.2002**

(51) Int Cl.[7]: **C12Q 1/68**, C12Q 1/02, C12N 15/09, G01N 33/53, G01N 33/566, A61M 1/18

(86) International application number:
**PCT/JP2002/007896**

(87) International publication number:
**WO 2003/014394 (20.02.2003 Gazette 2003/08)**

(84) Designated Contracting States:
**AT BE BG CH CY CZ DE DK EE ES FI FR GB GR IE IT LI LU MC NL PT SE SK TR**
Designated Extension States:
**AL LT LV MK RO SI**

(30) Priority: **03.08.2001 JP 2001236521**
**03.08.2001 JP 2001236522**

(71) Applicant: **ASAHI MEDICAL Co., Ltd.**
**Tokyo 101-8482 (JP)**

(72) Inventors:
- **SOUKA, Takayuki**
  **Fuji-shi, Shizuoka 416-0939 (JP)**
- **OHNO, Takeshi**
  **Fuji-shi, Shizuoka 416-0955 (JP)**
- **MANIWA, Shunji**
  **Nobeoka-shi, Miyazaki 882-0866 (JP)**
- **SATOH, Jun-ya**
  **Nobeoka-shi, Miyazaki 882-0856 (JP)**

(74) Representative:
**Forstmeyer, Dietmar, Dr. rer. nat., Dipl.-Chem. et al**
**Boeters & Bauer,**
**Bereiteranger 15**
**81541 München (DE)**

(54) **METHOD OF EVALUATING BIOCOMPATIBILITY**

(57) A method of evaluating the biocompatibility of a medical device (mainly a material to be in contact with blood). An aqueous nucleated cell-containing solution such as collected blood is brought into contact with the surface of a material for a definite period of time in vitro and then the cell-containing liquid is collected. Next, an RNA fraction is extracted from the cells and cDNA are constructed with the use of reverse transcriptase. Then the expression level of a specific gene is quantified by the real time PCR method or the DNA array method. Separately, the above procedure is repeated but not bringing into contact with the material and the expression level of the specific gene in the cells is quantified. Then the expression levels thus quantified are compared with each other. A material showing a former expression level closer to the latter level has the higher biocompatibility. Using this method, a functional change in cells can be conveniently, quantitatively and highly sensitively evaluated in vitro, compared with the conventional methods. By comparing changes in expression level of urokinase plasminogen activator receptor gene or urokinase plasminogen activator receptor protein, a functional change in cells can be evaluated from a viewpoint available in none of theconventionalmethods. By regulating endotoxin contamination, the true change in cells by the material per se can be evaluated. Materials for treating a body fluid having been evaluated by the above method; a substance for evaluating biocompatibility; and a method.of selecting an evaluating marker.



Printed by Jouve, 75001 PARIS (FR)

## Description

Technical Field

The present invention relates to a method of evaluating biocompatibility for minimizing or eliminating an injurious physiological response, for example, a functional change in leukocytes such as detrimental physiological reactions including the activation of leukocytes and the suppression of the immune system, mainly in a medical device to be in contact with blood, particularly an extracorporeal circulation-type medical device. More specifically, the present invention relates to a method of evaluating the cell stimulatory of a medical material by quantifying a change in expression of a cytokine chemokine gene, a urokinase plasminogen activator receptor gene, a urokinase plasminogen activator receptor protein, or the like of leukocytes being in contact with the surface of a material at high sensitivity in a simple manner. Furthermore, the present invention relates to a material for treating a body fluid, which is evaluated by such an evaluation method, a substance for evaluating biocompatibility, and a method of selecting an evaluation marker.

Prior Art

In recent years, medical devices to be in contact with blood, particularly extracorporeal circulation-type medical device, have progressed remarkably. Thus, the number of troubles, such as the impossibility of continuing the extracorporeal circulation due to the occurrence of blood clotting or the like in extracorporeal-circulating operation, has decreased. The principle trouble at the time of an extracorporeal circulation treatment typified by hemodialysis is caused when a biological reaction is initiated by the contact between the surface of a material recognized as foreign matter and the blood. Main clinical manifestations include a variety of manifestations such as blood clotting, an anaphylatic shock, a transient decrease in leukocytes (leukopenia), pyrexia, and a decrease or elevation in blood pressure. Mechanisms of causing problems in extracorporeal circulation with those acute clinical manifestations have been studied for a long time. The causes of the manifestations have been thought to be the activation of platelets by bringing the blood into contact with the surface of a material, the activation of complement components in the blood by contacting with the surface such as cellulose having many hydroxyl groups, the production of bradykinin by bringing the blood into contact with a negative charged surface, a reversed flow of endotoxin from a dialysate to the blood, and the like. Considering those mechanisms, the conventional medical devices have improved the problems by improving the biocompatibility or blood compatibility of a material.

In recent years, however, there are a prolonged period of dialysis and aging of patients in the field of dialysis. Symptoms such as dialysis amyloidosis, increased susceptibility to infection, and arterial sclerosis, which are caused by repetitive dialysis treatments for long terms, have been seened as problems, so that further improvements have been desired. It is thought that those symptoms are closely related to the biocompatibility of materials for medical devices. However, in the conventional evaluation method, the biocompatibility could not be evaluated in an excellent manner enough to predict clinical manifestations in long terms, and the evaluation had to be performed by actually collecting the blood from a patient being subjected to dialysis. Thus, the evaluation could not be performed unless the safety and the performance were satisfied. From those reasons, there is an industrial requirement for the development of an evaluation method capable of extracorporeally evaluating multiple samples each having a small amount and capturing a slight alteration of a living body due to contact with a material.JP 07-178166 A discloses the evaluation of residual blood in a hollow fiber in the extracorporeal circulation of a rabbit and a method of measuring the active oxygen species discharged from leukocytes by converting the active oxygen species into luminescence. The method includes: bringing a suspension of leukocyte phagocytic cells isolated from human blood into contact with a material in the shape of a flat film; and adding 0.1 mM of 2-methyl-6-(-p-methoxyphenol)-3,7-dihydroimidazo(-1,2-a)-pyrazine-3-on (hereinafter, referred to as MCLA). However, the method is for evaluation under the condition where a large amount of active oxygen be discharged from leukocytes, so that it is difficult to make a comparison between materials each having a small production of active oxygen. In addition, the MCLA has problems such as a tendency to produce a false positive reaction by reacting with some chemicals and the surface of a material or auto-oxidation. JP 09-510903 A describes the examples in which measured are various markers such as thrombin-antithrombin III (TAT) of the blood contacted with a material, kallikrein-like activity, a clotting time, terminal complement complex (TCC activity), the amount of IL-1βprotein producction, the discharge of fine particles from platelets, the expression level of p-selectins, the amount of platelet-leukocyte aggregate and the expression level of L-selectins / CD11b on the leukocyte surface. Among them, the amount of IL-1β protein secretion, the amount of platelet-leukocyte associations, and the expression level of L-selectins / CD11b on the leukocyte surface can be used as markers for the functions of leukocytes in the present invention. There is reported that the IL-1β protein can be discharged when mononuclear cells (monocytes and lymphocytes) are brought into contact with the surface of a material (Cardona, M. A., et al., Biomed. Mater. Res. 26: 851-859 (1992)). However, the secretion of IL-1β protein is trace amounts. Thus, when the activation of leukocytes by the surface of a material is evaluated, effective detection cannot be performed unless a cellular activating agent such

as LPS is used (for instance, about 230.4 pg/ml in maximum for TNFα at a compound stimulus of a material + LPS for 18 hours). In addition, it is difficult to grasp the adsorption of IL-1β protein on the surface of a material and thus there is a problem in that a measured value may be calculated low depending on the material to be brought into contact with. It is always difficult to estimate the difference between the materials clearly based on the amount of platelet-leukocyte associations or the expression level of L-selectins / CD11b on the leukocyte surface.

In MEMBRANE 19 (6): 400-410 (1994) and "Novel High Performance Dialyzer for Hemodialysis Staff" (Tokyo-Igakusha, Co., Ltd.): 13-27 (1998), Sasaki and others have measured the secretion amount of each of proteins IL-1β, IL-6, TNFα, and granulocyte elastase and the amount of active oxygen production by luminescence using MCLA as markers for the compatibility of leukocytes when the proteins are brought into contact with a material. According to this method, it is difficult to accurately determine the secretion amount of a protein (particularly, the trace amount of the protein, for example a cytokine such as IL-1β, IL-6, or TNFα) because of the adsorption of the protein on the surface of a material as described above. In addition, there is a possibility of failing in detection of the slight activation of leukocytes below the detection limit of the measurement method. Furthermore, the detection is difficult unless the blood is left in prolonged contact with the material (for instance, less than 800 pg/ml in maximum for IL-1β under a 6-hour stimulus with the material and less than 200 pg/ml in maximum for TNFα under a 1-hour stimulus with the material, and it is equal to or less than the detection as in the case of completely free of stimulation depending on the kind of the material).

S. Kushihata et al. , Int. J. Artif. Organs 21 (7) : 384-390 (1998) describes a method of quantifying mRNA of TNFα in human mononuclear, which are brought into in-vitro contact with a hollow fiber material, by an RT-PCR method. In this case, however, the blood of an actual dialysis patient is used and the dialysis patient lacks erythropoietin responsible for erythrocyte production because of degradation in renal function. Thus, in many cases, the patient presents anemia. In addition, there is a problem in that a large volume of blood is hardly collected for the evaluation of leukocyte compatibility, so that the industrial application is limited remarkably. Furthermore, a PCR product after the RT-PCR is electrophores ed on an agarose gel and stained with EtBr. Then, a band of the PCR product is quantified as an image. Therefore, the operation is complicated and requires much skill, and thus there is a problem in that an accident error derived from the PCR reaction is too large to be analysed quantitatively. N. Weber et al., 2001 SOCIETY FOR BIOMATERIALS 27th Annual Meeting Transactions, page 185 describes a method including: bringing human blood into in-vitro contact with a tubular material; isolating monocytes from the blood; and quantifying mRNA of the monocytes after the contact with the material using an RT-PCR method. However, the operation of isolating the monocytes using magnetic beads on which anti-CD14 antibodies are fixed is complicated, so that the biocompatibility of leukocyte fractions other than the monocytes cannot be evaluated. Furthermore, as with the above description, a PCR product after the RT-PCR is electrophoresed on an agarose gel and stained with EtBr, and then a band of the PCR product is quantified as an image. Thus, the operation is complicated and requires much skill, and an accident error derived from the PCR reaction is too large to be analyzedquantitatively. Besides, the publication also describes a method of detecting the PCR product by a Southern blot method. However, the accident error derived from the PCR reaction cannot be dissolved by such amethod. From the above problems,anindustrially simplified quantitative method for evaluation of biocompatibility with high sensitivity has been requested.

In a recent study, the detection of a subtle change in a living body caused by the contact with a material has become possible. However, such a change in the living body in contact with the surface of the material cannot be explained entirely by only those markers. More markers are required for the evaluation of biocompatibility.

For finding out markers for biocompatibility other than those described above, the following has been performed. For example, a specific protein, which is quantitatively and qualitatively different between a dialysis patient and a healthy donor, is detected and then changes in the mass of the protein in the presence or absence of contact with the surface of a material are quantified by ELISA. Alternatively, changes in its volume on the cell surface are quantified with a flow cytometry or the like. Alternatively, changes in the genetic expression level of its gene is quantified. However, it is very difficult to find out a new marker because of the time-consuming and a large amount of labor.

Furthermore, in general, a blood cell has a high sensitivity to endotoxin (the terms "pyrogen" and "LPS" are also used in the same meaning) . Unless the evaluation on the gene expression level in the present invention is performed under conditions in which endotoxin is extremely limited in amount, the endotoxin with which a sample and an device are contaminated influences measurements significantly. In this regard, there is almost no description about endotoxin in the prior art documents described above. Even though the description about endotoxin is found, the endotoxin is only provided as a cell-activating agent to be added after material contact without considering the influence of the endotoxin contamination on the gene expression. Therefore, there is a problem in that the true biocompatibility of the material itself cannot be evaluated.

Disclosure of the Invention

An object of the present invention is to provide a novel marker for biocompatibility with which activation of

cells brought into contact with the surface of a material can be detected, which is hardly detected by the conventional method. Another object of the present invention is to provide a versatile and high-sensitive method of quantitatively evaluating biocompatibility of a material in a simple manner, the method not being limited to using the blood of a clinical subject or the like. In addition, another obj ect of the present invention is to provide a method of evaluating true biocompatibility of a material itself by regulating endotoxin contamination. A further object of the present invention is to provide a biocompatible material or a material for treating a body fluid, which is more excellent than the conventional one, by evaluating with the biocompatibility-evaluating method according to the present invention.

The inventors of the present invention have made an extensive study with a view to overcoming the problems and found out the fact that a functional change in leukocytes and the activation of the leukocytes can be quantified more simply and sensitively than the conventional method by: keeping a solution that contains a nuclear cell originating from a blood sample or a leukocyte-containing aqueous solution into in-vitro contact with the surface of a material for a given length of time; collecting the cell-containing solution or the leukocytes, followed by extracting an RNA fraction from the cells; preparing a cDNA from the extract by using a reverse transcriptase; quantifying the expression level of a specific gene using a real-time PCR method or a DNA array method; and comparing the measured expression level with the expression level of a specific gene of leukocytes obtained by repeating the same procedure except no contact with the material. Furthermore, the inventors have found out a surprising knowledge that an increase in expression level of a urokinase plasminogen activator receptor gene is caused by bringing the gene into contact with the material, the gene not having been known as a biocompatibility marker in the prior art. The inventors of the present invention have made a further study and found out that the density of urokinase plasminogen activator receptors on the cell surface is also increased by bringing the receptors into contact with the surface of the material. Besides, when the density of urokinase plasminogen activator receptors on the cell surface is used as a marker, under a certain measurement condition, the inventors have found a surprising knowledge that a material is excellent, the surface density of which after the contact with the material increases to be less than 1.80 times compared with the surface density having no contact with the material.

Furthermore, the present invention has been completed by finding out the fact that a true functional change in leukocytes by a material itself, particularly the activation of the leukocytes can be detected by regulating endotoxin contamination.

In other words, the invention pertains to a method of evaluating the biocompatibility of the surface of a material, a material for treating a body fluid, a substance for evaluating biocompatibility, and a method of selecting an evaluation marker.

(1) A method of evaluating biocompatibility, including: bringing the surface of a material and an aqueous solution containing a leukocyte into contact with each other in vitro for a definite period of time; recovering the aqueous solution containing the leukocyte; extracting an RNA fraction from the inside of the leukocyte; preparing cDNA from the extracted solution using a reverse transcriptase; quantifying the expression level of a specific gene using a real-time PCR method or a DNA array method; and comparing the expression level with the expression level of a specific gene of a leukocyte, which is similarly treated without being brought into contact with the material, to evaluate biocompatibility of the material.

In the present invention, it is judged that a material having a substantially no difference in the comparison between the gene expression level of the former gene and that of the latter gene is suitable material with biocompatibility, and is used as a material for a medical device such as a material for artificial kidneys.

(2) A method of evaluating biocompatibility, including: bringing the surface of a material and a nuclear cell-containing solution derived from an animal including a human being into contact with each other for a definite period of time; recovering the solution containing the cells; extracting an RNA fraction from the inside of the cells; preparing cDNA from the extracted solution using a reverse transcriptase; quantifying the expression level of a urokinase plasminogen activator receptor gene; and comparing the expression level with the expression level of a urokinase plasminogen activator receptor gene from nuclear cells derived from an animal including a human being, which are similarly treated without being brought into contact with the material.

In the present invention, it is desirable to quantify the expression level of the urokinase plasminogen activator receptor gene by means of a real-time PCR method or a DNA array method.

(3) A method of evaluating biocompatibility, including: bringing the surface of a material and a nuclear cell-containing solution derived from an animal including a human being into contact with each other for a definite period of time; recovering the solution containing the cells; quantifying the expression level of a urokinase plasminogen activator receptor protein on the surface of the cells, the intracellular level of the urokinase plasminogen activator receptor protein, or the extracellular level of a soluble urokinase plasminogen activator receptor protein; and comparing the quantified one with the expression level or the level of a urokinase plasminogen activator receptor protein of nuclear cells derived from an animal including a human being, which are similarly treated without being brought into contact with the material.

In the present invention, the material is evaluated as a suitable material material with biocompatibility as far as the quantative value of the expression level of the urokinase plasminogen activator receptor gene, of the cell-surface expression level of the urokinase plasminogen activator receptor protein or the intracellular level of the protein, or of the extracellular level of the soluble urokinase plasminogen activator receptor protein of the former has no substantial difference from the quantitative value of the latter.

According to the present invention, there is provided a material for treating a body fluid characterized in that the cell-surface expression level of a urokinase plasminogen activator receptor protein is less than 1.80 times, when the human peripheral blood is brought into contact with the surface of a material, compared with the cell-surface expression level of a urokinase plasminogen activator receptor protein in the human peripheral blood subjected to the same procedure without being brought into contact with the material, under the conditions in which a contact surface area ratio between the human blood and the material is 250 $cm^2$/ml to 160 $cm^2$/ml at 37°C for 30 minutes.

Furthermore, the present invention refers to a method of selecting an evaluation marker with respect to the cell stimulatory of such a substance and material for evaluating biocompatibility.

Hereinafter, the invention is described in detail.

The term "*in vitro*" ("in vitro" is used for the same meaning) described herein represents a process or a reaction caused in an artificial environment such as in a tube or in an incubator, but not including a case of taking blood once out of the living body and then returning the blood into the living body again like a dialysis or an extracorporeal circulation treatment performs.

The term "leukocyte-containing aqueous solution" described herein means a blood sample prepared by adding an anticoagulant (heparin, citric acid, or EDTA (which is exemplified for explanation, but the anticoagulant is not limited thereto)) to human peripheral blood or mammal peripheral blood or means a solution prepared by isolating leukocytes from the blood sample with the use of a difference in specific gravity and suspending the leukocytes in an aqueous buffer solution (PBS (-), HBSS, physiological saline, serum, or plasma (which is exemplified for explanation, but the solution is not limited thereto)) having a physiological salt concentration and pH.

The term "nuclear cell-containing solution" means a solution prepared by suspending cultured cells originating from animals including human beings, which can be proliferated in vitro, in an aqueous buffer solution (PBS (-), HBSS, physiological saline, serum, plasma, or culture medium (which is exemplified for explanation, but the solution is not limited thereto)) having a physiological salt concentration and pH. Alternatively, the term means a blood sample prepared by adding an anticoagulant (heparin, citric acid, or EDTA (which is exemplified for explanation, but the anticoagulant is not limited thereto)) to human peripheral blood or mammal peripheral blood, or means a solution prepared by prepared by isolating leukocytes from the blood sample with the use of a difference of specific gravity and suspending the leukocytes in an aqueous buffer solution having a physiological salt concentration and pH. The leukocyte-containing aqueous solution is also included in the category of the nuclear cell-containing solution.

The term "leukocytes" described herein include all of classified groups including granulocytes, mononuclear cell, neutrophils, acidophils, basophils, monocytes, and lymphocytes, or the leukocytes may be purified to a specific cell group such as neutrophils.

The contact time between the surface of a material and the nuclear cell-containing solution is, but not specifically limited to, desirably 10 seconds or more in terms of the efficiency of contact with the material. In addition, for preventing the survival rate of cells from decreasing, the contact time is desirably 6 hours or less. Furthermore, for preventing the recovery rate of cells from decreasing with their adhesion to the surface of the material, it is more desirably less than 2 hours.

The contact surface area ratio between the surface of the material and the nuclear cell-containing solution is, but not specifically limited to, desirably 1 $cm^2$/ml or more in terms of the efficiency of contact with the material. For preventing the recovery rate of the cells from decreasing with their adhesion to the surface of the material, the contact surface area ratio is desirably 500 $cm^2$/ml or less. In addition, the surface of the material and the leukocyte-containing solution may be brought into contact with each other by any procedure of keeping in still standing, agitating, and a fluid state of the cell-containing solution. For daring to exemplify a more suitable condition, the contact surface area ratio is desirably in the range of 250 $cm^2$/ml to 160 $cm^2$/ml.

Any temperature is suitable for the invention at the time of making contact between the surface of the material and the nuclear cell-containing solution as far as the temperature is 45°C or less for preventing the survival rate of the cells from decreasing and is 0°C or more at which the aqueous buffer solution is in a liquid state. More desirably, the temperature is in the range of 15°C to 37°C in which physiological activities of the cells are vivid.

As an example of the material described herein, any material can be used, which is capable of forming an interface with an aqueous solution such as a solid surface insoluble to water or a water-containing hydrogel surface. For daring to exemplify, materials in the shape of a hollow fiber, filament, nonwoven fabric, filter, flat film, and particle are suitably used in the present invention. In addition, one prepared by applying a polymer material on the surface of the above material together with a solvent and volatilizing only the solvent therefrom to coat the polymer material on the surface, one prepared by subjecting the surface of a material to a surface modification with physical and chemical

methods to improve the solid surface, one prepared by graft polymerization of a polymer material on the solid surface, and so on may be suitable for the invention.

The term "RNA fraction" described herein means the total of RNAs (hereinafter, referred to as "total RNA") including all of ribosomal RNAs, transfer RNAs, and messenger RNAs (hereinafter, referred to as "mRNAs") and mRNAs purified using an oligo-dT column or the like. As a method of extracting the RNA fraction, but not specifically limited to, an Acid Guanidium-Phenol-Chloroform method (hereinafter, referred to as an "AGPC method") is preferably used in the invention in that the extraction can be attained in a simple manner with a small amount of the sample.

The primer described herein used for preparing cDNA using a reverse transcriptase is, but not specifically limited to, desirably an oligo-dT primer or a genetically specific antisense primer in terms of reducing the effects of ribosomal RNAs.

The ream-time PCR method described herein includes: converting the amount of a PCR product into the fluorescence intensity; recording the intensity every one cycle of the temperature cycle of the PCR to precisely identify the cycle of a logarithmic linear growth period in the PCR; and calculating the number of cycles enough to distinguish a signal such as the fluorescence intensity from the background thereof to determine the amount of cDNA before the PCR reaction (reflecting the amount of mRNA) . As a method of detecting the fluorescence, although there is no particular limitation, each of an intercalation method, a TaqMan prove method, a uniprimer method, and a hybrid-probe method can be suitably used in the present invention (as a reference, there is Kawaguchi et al., Journal of Medical Technology 44 (6), 2000).

The DNA array method described herein includes bringing two or more DNA sequences into line to be fixed on a substrate made of glass, a nylon film, or the like. Preparation and detection methods thereof include: a method provided from Affimetrix, Inc; and a method provided from Stanford University. However, the methods are not limited to the specific one, so that any method can be suitably used in the present invention (see, "Close-up Experimental Method" in Experimental Medicine, vol. 19 (2001) Nos. 1-8 (Yodosha, Co., Ltd.)). In those detection methods for quantification, an RNA fraction is labeled with a radio isotope or a fluorescent material at the time of preparing a cDNA fraction using a reverse transcriptase, or the RNA fraction is labeled with the radioisotope or the fluorescent material in advance after the preparation of the cDNA using the reverse transcriptase and is then hybridized with the DNA sequences being fixed in line. After the DNA sequences have been washed, the radioactivity or fluorescence intensity of a spot of each DNA sequence is measured. In the present invention, for example, there is another suitable method including: preparing two or more independently labeled cDNAs from two or more kinds of RNA fraction samples; and bringing the cDNAs into competition with each other while allowing the hybridization of the cDNAs. Those methods do not require the step of the PCR, so that the methods can be expected to be quantitative.

The cytokine described herein is a generic term for protein factors that exhibit a physiological activity through a specific receptor on a cell surface when it exists at an extremely small amount. Although all of those factors are preferably used in the present invention, the following are particularly exemplified: Interleukins (IL-1α, IL-1β, IL-2, IL-3, IL-4, IL-5, IL-6, IL-7, IL-8, IL-9, IL-10, IL-11, IL-12, IL-13, IL-14, IL-15, IL-17, IL-18, etc.), an Interleukin-1 receptor antagonist, Human B cell growth factor-12kD (BCGF-12kD), CD40 Ligand (CD40L) , Tumour necrosis factor (TNFα, TNFβ), interferons (INFα, INFβ, and INFγ), Leukaemia inhibitory factor (LIF) , Oncostatin M (OSM) , Ciliary neurotrophic factor (CNTF), Nerve growth factors (NGF, BDNF, NT-3, and NT-4/5), Neu differentiation factor (NDF), Neuregurin (NRG), Erythropoietin (EPO), Granulocyte colony-stimulating factor (G-CSF), Colony-stimulating factor-1 (CSF-1), Macrophage colony-stimulating factor (M-CSF), Granulocyte-Macrophage colony-stimulating factor (GM-CSF), Stem cell factor (SCF), Hepatocyte growth factor (HGF), Epidermal growth factor (EGF) , Platelet-derived growth factor (PDGF), Insulin-like growth factor (IGF) , Fibroblast growth factor (FGF) , Transforming growth factor-β (TGFβ), Vascular endothelial growth factor (VEGF) , and Endothelin; and receptors thereof. In addition, when the factors are used for any animal blood, homologues of those are also preferred in the present invention. More preferably, IL-1α, IL-1β, IL-6, IL-8, and TNFα are preferred in the present invention. The chemokine described herein is a generic term for intrinsic leukocyte chemotactic factors. Although all of those factors are preferably used in the present invention, the following are particularly exemplified: GROα, GROβ, GROγ, PF4, ENA-78, GCP-2, NAP-2, IL-8, Mig, IP-10, I-TAC, SDF-1, BLC, BRAK, Lymphotactin, SCM-1β, Fractalkine, I-309, MCP-1, MCP-2, MCP-3, MCP-4, MIP-1α, MIP-1β, RANTES, Eotaxin, HCC-1, HCC-2, HCC-4, TARC, DC-CK-1, MIP-3α, MIP-3β, 6Ckine, MDC, MPIF-1, MPIF-2, TECK, Eotaxin-3, and CTACK (alias names of those factors include CXCL1 to CXCL15, XCL1, XCL2, CX3CL1, and CCL1 to CCL27 (reference: Albert Zlotnik et al., Immunity 12 (February) 121-127 2000)) ; and receptors thereof such as CXCR1 to CXCR5, XCR1, CX3CR1, and CCR1 to CCR10. In addition, when the factors are used for any animal blood, homologues of those are also preferred in the present invention. More preferably, MCP-1 and IL-8 are preferred in the present invention.

The term "urokinase plasminogen activator receptor" (hereinafter, referred to as "uPA-R") described herein has another name of monocyte activation antigen 3 (hereinafter, referred to as "Mo3") or CD87 (hereinafter, "uPA-R", "Mo3", and "CD87" represent a similar molecule). A function of the receptor is to express the serine protease activity thereof by converting plasminogen into plasmin by the action of the plasminogen activator bound to the uPA-R. The

plasmin activity is localized near the surface of tumor cells or cells related to inflammation. This molecular localization appears in leukocytes including mononuclear phagocytes, neutrophils, and T-cells, endothelial cells, liver cells, and some hematopoietic or non hematopoietic tumor cells. The human CD87 molecule is a glycoprotein having a molecular weight of about 50 to 60 kD and is a surface antigen appeared on the surface of a cell membrane such that a glycosylphosphatidyl inositol (GPI) anchor bonds with glycine at position 283. In addition, the soluble CD87 molecular species are found in myeloblast cell strain U-937 stimulated with an inflammation mediator, the culture medium of monocytes, and the blood or body fluid of a patient suffering from inflammation or tumor (as a reference, there is Uda et al., a separate volume of IGAKU NO AYUMI, "Handbook of CD Antigen", Ishiyaku Publishers, Inc., pages 439-442, 1999).

It is reported that, in the U-937 cell strain, 8-hour stimulation with TNFα or IFNγ causes an increase in mRNA of uPA-R and 24-hour stimulation with IFNγ increases the expression level of uPA-R protein on the surface of U-937 cells, while no increase is caused by 24-hour stimulation with TNFα (Robert G. Sitrin et al., Blood, vol. 84, No. 4, pages 1268-1275, 1994).

It is reported that, in the peripheral blood of the normal human subject, 4 to 24-hour stimulation with LPS (1-1000 ng/ml) increases the expression level of uPA-R protein on the surface of monocytes and 4-hour stimulation with a comparatively high concentration of TNFα (1-100 ng/ml) (Pascale E. P. Dekkers et al., Infection and Immunology, pages 2156-2160, Apr. 2000).

It is reported that, in the keratinocyte (corneocyte) cell strain, each of 24-hour stimulation with a comparatively high concentration of TNFα (10 ng/ml) and 24-hour stimulation with IL-1β (500 U/ml) increases the expression level of uPA-R protein on the surface of the cells and also the stimulation with TNFα (10 ng/ml) or IL-1β (500 U/ml) allows an increase in mRNA of uPA-R after 1 or 2 hours (Michael J. Bechtel et al., Experimental Cell Research, vol. 223, pages 395-404, 1996).

In the method of evaluating biocompatibility of the present invention, any method of detecting and quantifying CD87 molecules on the cell surface and in the cells and the soluble CD87 molecules present in the nuclear cell-containing solution can be suitably used. For instance, a flow cytometer method, an ELISA method, or an RIA method, in which fluorescent labeled antibodies are used, are desirable in terms of quantitativity. As a method of utilizing a substance having affinity to a urokinase plasminogen activator receptor protein molecule (CD87 molecule), the amount or activity of the urokinase plasminogen activator and the amount or activity of plasmin to be produced by being activated by the urokinase plasminogen activator allow the detection and quantification of the CD87 molecule on the cell surface. Besides, the density of the urokinase plasminogen activator receptor on the cell surface can be also estimated by measuring the migration ability of the cells to the urokinase plasminogen activator. In the method of evaluating biocompatibility of the invention, any method of detecting and quantifying the gene expression level of the CD87 molecule after isolating the mRNA fraction from nuclear cells can be suitably used. For instance, a real-time RT-PCR method, a northern hybridization method, and a DNA array method are preferable in terms of quantitativity. In the case where animal blood or the like is considered, homologues (both proteins and genes) of human uPA-R molecules are also suitable for the present invention.

Substances for evaluating biocompatibility of the present invention, which are provided for detecting the urokinase plasminogen activator receptor gene, include nucleic acids which can be specifically hybridized or annealed on the urokinase plasminogen activator receptor gene. In terms of the specificity of the detection, desirable is a nucleic acid having 10 or more residues of the same or complementary sequence of cDNA or mRNA of the urokinase plasminogen activator receptor gene. Examples of the sequence are SEQ ID NOS: 1 and 2 in the present description (which are described for the description, but the sequence is not limited thereto). With regard to the above nucleic acid, a nucleic acid containing a single-stranded DNA, a double-stranded DNA, a single-stranded RNA, and a label for the detection, and also derivatives of the nucleic acid are also suitable for the present invention.

Substances for evaluating biocompatibility of the present invention, which are provided for detecting the urokinase plasminogen activator receptor protein molecule, include substances having affinity to the urokinase plasminogen activator receptor protein molecule (CD87 molecule). As for the affinity of this case, the dissociation constant with a urokinase plasminogen activator receptor protein molecule in an aqueous solution is desirably Kd = $10^{-7}$ M or less in terms of quantitativity and specificity. Daringto say, examples of the substances having affinity to the CD87 molecule include antibodies for the protein molecules of urokinase plasminogen activator and urokinase plasminogen activator receptor. It is also possible to label the antibodies with an enzyme, fluorescence, and radioisotope. The CD87 R-PE-conjugated mouse anti-human monoclonal antibody: U. S. Pharmingen Inc., in the present description is one of the examples (which is described for explanation, but the antibodies are not limited thereto).

The term "cell stimulatory of a medical material" described herein means that the expression of some kinds of genes or proteins is enhanced or diminished when cells are brought into contact with the surface of a material directly or indirectly through plasma components or the like, compared with the expression at the time of keeping out of contact with the material.

The term "causing no substantial difference" described herein means that there is no significant difference when analyzed statistically.

The term "biocompatibility" described herein means the nature with which harmful action is not exerted to a living body when the living body is brought into contact with the surface of a material directly or indirectly through plasma components or the like. Specific examples illustrated daringly include the nature with which an artifact transplanted in the body is hardly refused by an immune reaction or the like. In this case, the term "good biocompatibility" means that there is a minimum or no harmful physiological reaction such as the activation of leukocytes, compared with other materials.

The term "body fluid" described herein means a liquid that is present in the living body and that contains water as a main component, cellular components, solid components, and oils. Examples of the body fluid illustrated daringly for explanation include, but not limited to, blood, a bone marrow fluid, ascites, pleural effusion, lympha, urine, bile, a digestive juice, a tissue fluid, mucin, perspiration, and an interstitial fluid.

The term "treatment on a body fluid" described herein means that some kinds of action such as contact with the surface of a material, a temperature change, and a pressure change are artificially effected on the living body *in vivo, in vitro* or *ex vivo*. Examples of the treatment illustrated daringly for explanation include, but not limited to, the extracorporeal circulation of blood, blood sampling, transfusion, bone marrow sampling, bone marrow biopsy, and ascites sampling. In addition, when a material insoluble in water or a material that forms an interface with water is embedded or implanted in a given place in the body, the tissue of the living body can be brought into contact with the material through a tissue fluid, lympha, blood, an interstitial fluid, or the like. Thus, this case also corresponds to the treatment on a body fluid.

The term "material for treating a body fluid" described herein means a material that does not generate a substantial difference in expression level of a specific gene, compared with the gene expression level of cells subjected to similar procedures except that the cells are kept out of contact with the material, when the evaluation is performed using the method of evaluating biocompatibility of the present invention.

In other words, the term means a material for treating a body fluid that does not cause a substantial difference compared with the gene expression level of the urokinase plasminogen activator receptor molecule in nuclear cells subjected to similar procedures except that the cells are kept out of contact with the material, the expression level of the urokinase plasminogen activator receptor protein on the surface of the cells, the concentration of the urokinase plasminogen activator receptor protein in the cells, or the concentration of the soluble urokinase plasminogen activator receptor protein of other than nuclear cells in the nuclear cell-containing solution.

Among them, in the case of the urokinase plasminogen activator receptor protein, when the human peripheral blood is brought into contact with the surface of a material at 37 °C for 30 minutes with a contact surface area ratio between the blood and the material in the range of 250 $cm^2$/ml to 160 $cm^2$/ml, the expression level of the receptor protein on the cell surface must be less than 1.80 times as high as the expression level of the urokinase plasminogen activator receptor protein of the human peripheral blood subjected to the similar procedures but not contact with the material. If the expression level of the urokinase plasminogen activator receptor protein on the cell surface becomes 1.80 times or more as high as that of the comparative control, the cells are in a highly activated state. Thus, it is not preferable because of easily inducing an inflammation reaction. A mechanism by which the inflammation reaction is included is considered to be as follows. The higher the expression level of the urokinase plasminogen activator receptor protein on the cell surface, the more the number of urokinase plasminogen activators (hereinafter, referred to as uPAs) which are ligands of the protein binding on the surface of the cells. As a result, the plasminogen present in the blood is converted in the neighborhood of the cell surface into plasmin which is a protease. More plasmin activity can be localized in the neighborhood of the cell surface. Finally, the ability of the activated cells to permeate through the tissue increases extensively. The more preferable range of the expression level is less than 1.76 times, particularly preferable 1.72 times or less. In addition, the lower limit of the numerical value preferable for the material for treating a body fluid is 1.00, which is equal to that of the comparative control. The above value can be theoretically less than 1.00. In this case, it is thought that nuclear cells are already in an activated state of while the cells are kept out of contact with the material. Thus, the evaluation as the material for treating a body fluid is not proper. However, when the above value is less than 1.00 by a degree that no significant static difference is recognized, the value is considered to be equal with that of the comparative control in the present description.

The material for treating a body fluid described herein may be any material as far as the material shows the biocompatibility, so that the raw material and configuration of the material are not specifically limited, and the material may be as a water-insoluble solid surface, a hydrogel surface, or the like as far as the material forms an interface with an aqueous solution.

Preferably, used at the time of treating a body fluid is a material which cannot be mechanically destroyed by the flow of a body fluid, a material which does not deform to a degree that the function of the material becomes impaired, or the like. Examples of such a material include a structural body prepared by molding a hydrophobic polymer and a hydrogel provided as a structural body in which hydrophilic polymers are cross-linked in a three-dimensional manner.

When the material is only constructed of a hydrophobic polymer, the material tends to activate the cells in general, thereby resulting in cell adhesion or the like. On the other hand, when the material is only constructed of a

hydrophilic polymer, insufficient points remains in the strength or handling as a material. Thus, a material mainly composed of a hydrophobic polymer and a hydrophilic polymer prepared by mixing the polymers is more preferable. Considering the strength thereof as a material for treating a body fluid, it is recommended that the percentage content of the hydrophobic polymer is 50% by weight or more per total, while the percentage content of the hydrophilic polymer is not higher than a desired level.

Furthermore, considering the fact that a body fluid is brought into contact only with the surface of the material, the category of the present invention includes: a given material coated with a specific polymer material; a solid having a surface modified by physical and chemical methods for improving the surface of the solid; and a solid having a surface on which a polymer material is graft-polymerized. Examples of the hydrophobic polymer described herein include most of engineering plastics such as polysulfone, polyethersulfone, polyamide, polyimide, polyphenylether, andpolyphenylene sulfide. Particularly preferable is polysulfone having a basic skeleton described below. In the basic skeletons described below, but not limited to, a basic skeleton having a modified benzene ring portion is preferably used.

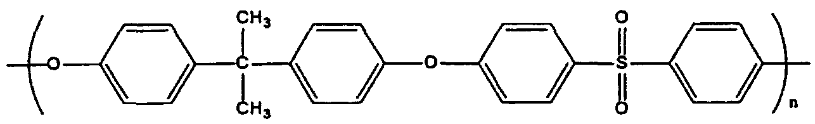

(I)

wherein n represents an integer number of 1 or more.

The term "polysulfone resin" described herein represents a derivative of the polysulfone skeleton shown in the above formula (I), polyethersulfone having the basic skeleton of the formula (II) in which diphenyl sulfone is ether bonded, and so on.

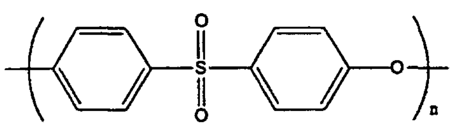

(II)

wherein n represents an integer number of 1 or more.

For the purpose of illustration, although there is no particular limitation, examples of the hydrophilic polymer described herein include polyethylene glycol, polypropylene glycol, polyvinyl alcohol, carboxymethylcellulose, and polyvinyl pyrrolidone, which may be used independently or in combination. In addition, a random copolymer, a block copolymer, a graft copolymer, or the like, which has both a hydrophilic polymer portion and a hydrophobic polymer portion in a molecule, can be also used suitably.

In the present description, polyethylene oxide (hereinafter, referred to as PEO) and polyethylene glycol (hereinafter, referred to as PEG) mean a similar molecular structure.

The form of the material for treating a body fluid is not specifically limited. For daring to exemplify, a material in the shape of a hollow fiber, filament, nonwoven fabric, flat film, or particle is suitably used in the invention. In the case of a material in the shape of hollow fiber, filament, or flat film, the structure of the material maybe of the so-called uniform structure in which polymer particles are tightly packed, a multi-pore structure like a sponge, or one of these structures provided with macrovoids. In the case of a material in the shape of a particle, the material may be of a multi-pore structure.

In addition, the function of the material for treating a body fluid is not specifically limited. However, for daring to exemplify, it is preferable for the material for treating a body fluid to have a dialyzing or filtrating function that shows selective permeability and/or an adsorbing function that shows selective adsorbability of the material.

Various combinations of those forms and functions can be used as a hollow fiber film, a non-woven filter, a non-woven adsorbent, a particulate adsorbent, and so on.

Specific examples of the "applications of a body fluid treatments" described herein include: the applications of blood treatments such as the extracorporeal circulation of blood, blood sampling, transfusion, and the production of blood preparations; and procedures such as bone marrow collection, bone marrow biopsy, and ascites tapping. The extracorporeal circulation of blood canbe subdivided into the applications of blood purification such as the exchange of plasma and adsorption therapy in addition to hemodialysis, hemofiltration, and hemodiafiltration.

The "material for treating a body fluid" described herein is suitable in any of those body fluid treatments. Most preferably, the material can be used for hemodialysis, hemofiltration, and hemodiafiltration, which are methods for

treating of chronic renal insufficiency. This is because the hemodialysis treatment is performed considerably frequently and continuously repeated for a long time, and thus in an application having a high frequency of stimulating a body fluid with the material, extremely excellent biocompatibility of the material is required.

A hollow fiber membrane is preferably used as a material for treating a body fluid, which can be used for the applications of thebloodpurification including the hemodialysis because of its advantages in compactness, high strength, and small blood volume. The inner diameter of the hollow fiber membrane may be 100 μm to 300 μm. Considering the thrombogenesis and the efficiency of exchanging materials during use, the inner diameter is preferably 160 μm to 250 μm. In addition, in terms of strength and handling properties, the thickness of the hollow fiber membrane is preferably 15 to 100 μm; when the thickness is larger than that range, the permeability of the material falls.

The "water pearmeability" described herein is a marker for the permeability when a material for treating a body fluid is used as a membrane. When pure water is filtrated through the membrane from one side to the other with a certain pressure applied thereto, the water pearmeability indicates a filtration volume of pure water flowing out to the other side per unit hour and unit area. For instance, in the case of the hollow fiber membrane, a module where one end of the hollow fiber membrane is sealed is prepared and then the filtration volume from the inside to the outside or from the outside to the inside of the hollow fiber membrane is measured, allowing the calculation of the water pearmeability with the following equation:

$$\text{Water pearmeability } (ml/m^2 \cdot mmHg \cdot hr) = Qw / (P \times T \times A)$$

Here, Qw denotes a filtration volume (ml), T denotes an efflux time (hr), P denotes a pressure (mmHg), and A denotes a membrane area ($m^2$: on the basis of the inner surface of the hollow fiber), respectively.

An excessively low water pearmeability of pure water is unsuitable for dialysis and filtration because the water cannot be substantially permeated, the pore tends to be small, and thus the permeability of a solute becomes deteriorated. In contrast, if the permeability is too high, for example, albumin which is one of the useful materials in a body fluid tends to be freed. Therefore, the water pearmeability is preferably ranging from 5 $ml/m^2 \cdot mmHg \cdot hr$ or more to 1000 $ml/m^2 \cdot mmHg \cdot hr$ or less.

Furthermore, the term "β2-microglobulin" described herein (hereinafter, referred to as β2MG) means a protein with a molecular weight of 11,800, which is noncovalently associated with a major histocompatibility antigen MHC class I molecule present on the cell surface and which is in a free state in blood or serum. In a state of renal insufficiency, the blood concentration of the β2MG is higher than that of the helthy donor and a tendency is recognized that it accumulates as the treatment is prolonged.

The sieving coefficient of β2MG is used as a marker for confirming an improvement in symptoms of the long-term dialysis subject particularly when the material for treating a body fluid is a hemodialysis membrane, a hemo filtrating membrane, or a hemodiafiltration membrane. The measuring and calculating methods of the sieving coefficient are described in "New High-Performance Dialyzer for Hemodialysis Staff" (Shingo Takezawa Ed., Tokyo Igakusha, Co., Ltd., pages 111-113, 1998) in detail.

The blood concentration of β2MG cannot be lowered in the dialysis treatment when the sieving coefficient of β2MG is too low. Thus, the sieving coefficient is preferably in the range of 0.4 or more and 1 or less.

The material for treating a body fluid is, considering the uses thereof, preferably sterilized to kill the microbial cells therein. In general, methods of sterilizing the material include dry sterilization, high pressure steam sterilization, gas sterilization using formalin gas or ethylene oxide gas, and radiation sterilization using an electron beam, γ-radiation, or the like. However, considering the damage on the material and the problem of remaining gas, or productivity, the radiation sterilization is preferable and most preferable is the γ radiation sterilization which is excellent in permeability to the inside of the material.

In addition, such a sterilization treatment is carried out in a dry state in which the material does not substantially contain moisture, or in a wet state in which the material contains moisture in the degree that the material does not drip off or the material is completely dipped in water. On that occasion, in terms of reducing the oxidative degradation of the material, it is preferable to sterilize the material in a wet state.

The cells used for the quantitative determination of the expression level of urokinase plasminogen activator receptor protein on the surface thereof are not specifically limited. However, there is a need of quantifying the cells that express the protein on the surface thereof by a degree not less than the detection sensitivity in advance, so that it is preferable to carry out the quantification on the surface of leukocytes when a nuclear cell-containing solution to be brought into contact with the surface of the material is the human peripheral blood. In addition, in terms of perceptively reflecting the degree of a contact stimulus with the material as a change in surface expression level of the protein, granulocytes and monocytes which are leukocytes excluding lymphocytes are preferable, and among them the monocytes are most suitably used in the present invention.

The "method of selecting an evaluation marker with respect to the cell stimulatory of a material" described

herein covers cases including changes in gene expression among various changes that occur in cells upon contact with the surface of the material, and those cells are compared with other cells not yet in contact with the material and among them, one showing a difference in comparison between the cells is provided as a candidate. It is preferable that a method of making such a comparison is one enabling comparison of plural kinds of genes at the same time. In addition, for selecting biocompatibility markers other than those known in the art, there is a need of examining numerous kinds of genes. Therefore, the method which can be suitably used in the present invention is a method including: extracting an RNA fraction from the cells; preparing cDNAs from this extraction solution using reverse transcriptase; and quantifying the expression level of a specific gene usinga DNAarraymethod. In addition, in the future, it is thought that the RNA fraction will be directly or quantitatively amplified and used to allow cyclopaedical expression analyses such as the DNA array method. Those methods can be applied to the method of selecting an evaluation marker for the cell stimulatory of the material described herein.

A routine evaluation on the biocompatibility of the material using the DNA array method is limited to some of facilities having special equipments, and requires additional costs. On this account, a utility value of the evaluation marker for the cell stimulatory of the material will spread if there is performed an operation that confirms the presence of a difference in expression levels of the candidate gene based on whether or not the candidate gene is in contact with the material by another method of quantifying the gene expression other than the DNA array method. As a quantative determination of the gene expression of this case, but not specifically limited to, a real-time PCR method is the most preferable in terms of quantitativity and simpleness.

In addition, the difference in expression levels of the candidate gene will finally lead to a difference in expression levels or concentrations of a protein coded by the candidate gene. Therefore, the evaluation markers for the cell stimulatory of the material can be used for general purposes if the operation for confirming the presence of a difference in gene expression levels of the candidate gene based on whether or not the candidate gene is in contact with the material is performed by quantifying the expression levels or concentrations of the protein coded by the candidate gene.

## Brief Description of the Drawings

Fig. 1 is a diagram that compares the expression level of a uPA-R protein molecule on the surface of granulocytes kept in contact with the surface of a material to the expression level of a uPA-R protein molecule on the surface of the granulocytes not yet in contact with the surface of the material.

Fig. 2 is a diagram that compares the expression level of a uPA-R protein molecule on the surface of monocytes kept in contact with the surface of the material to the expression level of a uPA-R protein molecule on the surface of the monocytes not yet in contact with the surface of the material.

## Best Mode for carrying out the Invention

Hereinafter, the present invention will be described in more detail with regard to the examples and the comparative examples. However, the present invention is not limited thereto.

(Example 1)

All of the devices used for the experiment were pyrogen-free up to the step of cell lysis to avoid endotoxin contamination.

From the elbow vein of the healthy donor, 100 ml of blood was drawn with a disposable syringe and a 19G winged needle. Then, the blood was gently transferred into a 200-ml PP container (polypropylene, 2075 Falcon Co., Ltd. , U.S.A.) containing 500 "1 of a heparin sodium solution (Shimizu Pharmaceutical, Co., Ltd., Japan) without any delay (final heparin concentration: 5 U/ml) , and the whole was then mixed gently. The blood was gently mixed with a sterilized physiological saline solution of dextran T-70 at a ratio of 9 : 1, and the mixture was left standing at room temperature for 40 minutes. From the upper portion of the mixture, the leukocytes and platelet-rich plasma (hereinafter, referred to as "LPRP"), was collected, and then the number of leukocytes was adjusted to 1 x $10^7$ per ml using the platelet-poor plasma derived from the same blood-supplying subject (hereinafter, referred to as "PPP").

AM-SD-150U (manufactured by Asahi Medical Co., Ltd., Japan) was used as a hollow fiber material. After the inner and outer surfaces of a hollow fiber had been washed with 500 ml of a physiological saline solution (Otsuka Pharmaceutical Co., Ltd., Japan) in a clean bench, AM-SD-150U (made of a recycled cellulose film, hereinafter referred to as "AM-SD") was aseptically decomposed by a dialyzer, followed by removing out the hollow fiber to provide a sample material under test.

The hollow fiber material was aseptically cut into pieces in a clean bench with an external surface area of 80

$cm^2$ and placed in 50-ml PP tubes (polypropylene, 2070 Falcon Co., Ltd., U.S.A.), followedby washing three times with 20 ml of a physiological saline solution. Then, 9 ml of the LPRP was added to each of them (material: LPRP-contact surface area ratio 8.9 $cm^2$/ml) and each resultant was then brought into contact with the material in a water bath (Thermo Minder DX-10, manufactured by Taitec Co., Ltd., Japan) at 37°C for 15 minutes, while reverse-mixing occasionally. As a comparative control, the same procedure was performed without the addition of the hollow fiber material. LPRP after contact with the material was transferred into a 50-ml PP tube (polypropylene, 2070 Falcon Co., Ltd., U. S.A.) and was then incubated in an incubator (NIB-90, manufactured by Iwaki Co. , Lid. , Japan) at 37°C for 60 minutes. The cells were centrifuged at 4°C by 400 g for 10 minutes and was then subjected to washing operations two times with resuspending in 10 ml of a physiological phosphate buffer solution (hereinafter, "PBS(-)").

A cell pellet obtained by removing the PBS(-) after centrifuging the cells was transferred into a 1.5-ml PP tube (polypropylene, Safe Seal Microcentrifuge Tubes, manufactured by Sorenson Bioscience, Inc), followed by dissolving the cells with 1 ml of Trizol (manufactured by Gibco BRL Co. , Ltd. , U.S.A.) and extracting the total RNA from the solution as described in the instruction manual. Each sample of total RNA (20 "g) was labeled with Cy5 or Cy3 (manufactured by Amersham Pharmacia Co., Ltd.) to prepare cDNA labeled with Cy5 or Cy3 using the Atlas™ Glass Fluorescent Labeling Kit (manufactured by Clontech Co., Ltd., U.S.A.) as described in the instruction manual thereof. At this time, it was designed such that cDNA derived from the cells not yet in contact with the material was different from cDNA derived from the cells after coming in contact with the material. The cDNA derived from the cells not yet in contact with the material and the cDNA derived from the cells in contact with a given material, which were labeled with different labels, were mixed with each other in equal amounts to allow the hybridization thereof overnight at 50°C as described in the instruction manual of the Atlas™ Glass Human 1.0 Microarray (manufactured by Clontech Co. , Ltd. , U.S.A.). Scaning data was obtained using the ScanArray Lite (manufactured by GSI Lumonics Co., Ltd., U.S.A.) and the Quant Array ver. 2.0 (manufactured by GSI Lumonics Co., Ltd. , U.S.A.) , and the conditions for obtaining the data were determined such that the fluorescence intensities of house keeping genes of the Cy5 corresponded with those of the Cy3 with a laser power of 80 V. Furthermore, for analyzing the data, the data was digitalized using the GeneSpring™ 3.0 (manufactured by Silicon Genetics Co., Ltd., U.S.A.).

In the case where a sample brought into contact with AM-SD was labeled with Cy5 and a sample not yet in contact with the material was labeled with Cy3, a fluorescence intensity ratio Cy5/Cy3 was 2.39 for the IL-1β gene and 4.14 for the IL-8 gene, respectively. It was found that, by being brought into contact with the AM-SD, the gene expression increased 2.39 times for the IL-1β and 4.14 times for the IL-8 , respectively. Therefore, the biocompatibility of the material was able to be quantified using the gene expression as a marker. Further, for confirmation, the fluorescent marker was replaced and the sample kept in contact with the AM-SD was labeled with Cy3. The fluorescence intensity ratio Cy3/Cy5 when the sample not yet in contact with the material was labeled with Cy5 was 3.13 for the IL-1β gene and 4.35 for the IL-8 gene, respectively, so that the same data was able to be obtained. In addition, in the case of the other genes, the condition of $0.5 < Cy5/Cy3 < 2$ was satisfied. In the case of labeling with replacing a fluorescent die, the condition of $0.5 < Cy3/Cy5 < 2$ was satisfied and a gene whose expression varies significantly could not be found.

(Example 2)

All of the devices used for the experiment were pyrogen-free up to the step of cell lysis to avoid endotoxin contamination. In addition, for confirming the quantitativity, the experiment was performed with n = 3.

From the elbow vein of the healthy donor, 60 ml of blood was drawn with a disposable syringe and a 19G winged needle. Then, the blood was gently transferred into a 200-ml PP container (polypropylene, 2075 Falcon Co. , Ltd., U.S.A.) containing 300 µl of a heparin sodium solution (Shimizu Pharmaceutical, Co., Ltd., Japan) without any delay, and the whole was then mixed gently, followed by being kept at room temperature until the start of the experiment (final heparin concentration: 5 U/ml).

APS-150S (manufactured by Asahi Medical Co., Ltd., Japan) and BK-1.6P (Toray Industries , Inc., Japan) were used as hollow fiber materials. Each of APS-150S (manufactured by blending polysulfone and polyvinyl pyrrolidone, hereinafter referred to as "APS") and BK-1.6P (prepared by blending isotactic polymethyl methacrylate and syndiotactic polymethyl methacrylate, hereinafter referred to as "BK") was provided as a sample material under test after removing out a hollow fiber by being aseptically decomposed by a dialyzer after washing the inner and outer surfaces of the hollow fiber with 500 ml of a physiological saline solution (Otsuka Pharmaceutical Co. , Ltd. , Japan). In addition, as for the BK, the sample under test was provided only as a hollow fiber prepared by inserting a thin fibrous spacer filament in the hollow fibers and aseptically isolating the thin fibrous spacer filament.

The hollow fiber material was aseptically cut into pieces in a clean bench with an external surface area of 40 $cm^2$ and placed in 15-ml PP tubes (polyprolylene,2327-015 Iwaki Co., Ltd., Japan), followed by washing three times with 5 ml of a physiological saline solution. Then, 5 ml of the previously collected blood was added to each of them (material: blood contact surface area ratio = 8 $cm^2$/ml) and each mixture was then set to a rotator "ROTATOR RT-5" (manufactured by Taitec Co. , Ltd. , Japan) , followed by contacting with each other while stirring at 2 rpm in an incubator

(NIB-90, manufactured by Iwaki Co., Ltd., Japan) at 37°C for 60 minutes. As a comparative control, the same procedure was performed without the addition of the hollow fiber material.

The blood after contact with the material was gently layered in a 15-ml PS tube (polystylene, 2321-015, manufactured by Iwaki Co., Ltd., Japan) containing 3 ml of a Ficoll-Paque solution (manufactured by Amersham Pharmacia Co., Ltd., U.S.A.) so as to form an interface therein, and the whole was then centrifuged by 400 g for 30 minutes at room temperature, followed by collecting a monocyte layer. Subsequently, 10 ml of a physiological saline solution was added and the whole was centrifuged by 400 g for 10 minutes at room temperature to obtain a washed monocyte pellet. The cell pellet was transferred into a 1.5-ml PP tube (polypropylene, Safe Seal Microcentrifuge Tubes, Sorenson BioScience, Inc.) , followed by dissolving the cells with 1 ml of Isogen (manufactured by Nippon Gene Co., Ltd., U.S.A.) and extracting the total RNA from the solution as described in the instruction manual. This total RNA (1 μg) was treated with DNase I (Amplification Grade) (manufactured by Gibco BRL Co., Ltd., U.S.A.) as described in the instruction manual, followed by adding Oligo dT (manufactured by Gibco BRL Co., Ltd., U.S.A.) therein to prepare cDNA using the Superscript II RT (manufactured by Gibco BRL Co., Ltd., U.S.A.) as described in the instruction manual. The cDNA thus prepared was diluted to a level of 5 ng/μl on the basis of the level of the total RNA being fed. Then, the cDNA was used as a template in a PCR reaction.

(Example 3)

The PCR template of Example 2 was used in the ABI PRISM 7700 Sequence Detection System (manufactured by Applied Biosystems Co., Ltd., U.S.A.) to carry out a real-time PCR quantification on the IL-6 gene. In addition, as an internal standard, the quantification of one of the house-keeping genes, the β-actin gene, was also carried out at the same time. The detection method used was a TaqMan probe method, where the Human IL-6 and the Human Beta-actin of Pre-Developed TaqMan Assay Reagents (manufactured by Applied Biosystems, Co., Ltd., U.S.A.), the TaqMan Universal PCR Master Mix (manufactured by Applied Biosystems, Co., Ltd., U.S.A.), and the MicroAmp Optical 96-Well Plate and Optical Caps (manufactured by Applied Biosystems Co., Ltd., U.S.A.) were used. A temperature cycle in PCR was 50°C for 2 minutes and then 95°C for 10 minutes, followed by 40-cycle repetition of two-temperature cycle of "95°C for 15 seconds and 60°C for 1 minute". An analysis on each sample normalized the expression level of the IL-6 gene in each sample by the expression level of the β-actin gene and then calculated the ratio of IL-6 / β-actin. Furthermore, the ratio was calculated as a relative value such that the IL-6 /β-actin value of the sample of the unstimulated material was defined as "1 (one)". The results are shown in Table 1.

(Example 4)

The real-time PCR quantification on the IL-6 gene was carried out using the PCR template of Example 2 in the LightCycler Quick System 330 (manufactured by Roche Diagnostics Co., Ltd., Germany). In addition, as an internal standard, quantification of one of the house-keeping genes, the β-actin gene and the G3PDH (GAPDH) genewascarriedout. Ahybridization probe method was used as a detection method for IL-6 and β-actin and the Light Cycler™ Fast Start DNA Master Hybridization Probes (manufactured by Roche Diagnostics, Co., Ltd. , Germany) was used. The SYBR Green I method was used as a detection method of G3PDH (GAPDH) and the Light Cycler™ Fast Start DNA Master SYBR Green I (manufactured by Roche Diagnostics, Co., Ltd., Germany) was used. Thefollowingwereusedasprimersandprobes: (SEQ ID NO. 1: 5'-AAGAGGCACTGGCAGAAAAC-3', SEQ ID NO. 2: 5'-GTCAGGGGTGGTTATTGCAT-3', SEQ ID NO. 3: 5'-CAGATTTGAGAGTAGTGAGGAACAAGCC-FITC-3', and SEQ ID NO. 4: 5'-LCRed-GAGCTGTCCAGATGAGTACAAAAGTCCT-Phosphorylated-3') for IL-6; (SEQ ID NO. 5: 5'-CCAACCGCGAGAAGATGAC-3', SEQ ID NO. 6: 5'-GGAAGGAAGGCTGGAAGAGT-3', SEQ ID NO. 7: 5'-CCTCCCCCATGCCATCCTGCGTC-FITC-3', and SEQ ID NO. 8: 5'-LCRed-GGACCTGGCTGGCCGGGACCTGA-Phosphorylated-3') for β-actin; and (SEQ ID NO. 9: 5'-TGAACGGGAAGCTCACTGG-3' and SEQ ID NO. 10: 5'-TCCACCACCCTGTTGCTGTA-3') for G3PDH(GAPDH).

Capillaries used for the PCR reaction were Light Cycler™ Capillaries (manufactured by Roche Diagnostics Co. , Ltd. , Germany). The concentration of $Mg^{2+}$ in a PCR reaction solution was 3 mM. The temperature cycle of the PCRwas (95°C for 10 minutes, followedby the 40-cycle repetition of three-temperature cycle, 95°C for 15 seconds, 55°C for 15 seconds, and 72°C for 11 seconds) for IL-6, (95°C for 10 minutes, followed by the 40-cycle repetition of three-temperature cycle, 95°C for 15 seconds, 59°C for 15 seconds, and 72°C for 19 seconds) for β-actin, and (95°C for 10 minutes, followed by the 40-cycle repetition of four-temperature cycle, 95°C for 10 seconds, 55°C for 5 seconds, 72°C for 13 seconds, and 85°C for 3 seconds) for G3PDH (GAPDH). An analysis on each sample normalized the expression level of the IL-6 gene in each sample by the expression level of the β-actin gene or the expression level of the G3PDH gene and then calculated the ratio of IL-6 / β-actin or IL-6 / G3PDH. Furthermore, each of the ratios was calculated as a relative value such that the IL-6 /β-actin or IL-6 / G3PDH value of the sample of the unstimulated material was defined as "1 (one)". The results are shown in Table 1.

(Comparative Example 1)

The PCR template of Example 2 was quantified using the conventional PCR method. In addition, as an internal standard, the quantification was performed on the G3PDH (GAPDH) gene, one of the house-keeping genes. The Taq polymerase (manufactured by Takara Shuzo Co., Ltd., Japan) was used as a DNA polymerase. Primers used were (SEQ ID NO. 11: 5'-ATGAACTCCTTCTCCACAAGCGCC-3' and SEQ ID NO. 12: 5'-GAAGAGCCCTCAGGCTGGACT-3') for IL-6 and (SEQ ID NO. 13: 5'-TGAAGGTCGGAGTCAACGGATTTGGT-3' and SEQ ID NO. 14: 5'-CATGTGGGCCATGAGGTCCACCAC-3') for G3PDH. The temperature cycle of the PCR was, for both genes, three-temperature cycle of 95°C for 45 seconds, 60°C for 45 seconds, and 72°C for 2 minutes, which was repeated 30 cycles. A 10-µl sample after the completion of the above PCR was electrophoresed on 1.5% agarose. After the sample had been stained with EtBr, a fluorescence image was obtained using the Gel Print 2000i/VGA (manufactured by Bio Image Co., Ltd., U.S.A.). The fluorescence image was quantified using the Basic Quantifier (manufactured by Bio Image Co., Ltd., U.S.A.) to quantify the PCR product. An analysis on each sample normalized the expression level of the IL-6 gene by the expression level of the G3PDH gene and then calculated the ratio of IL-6 / G3PDH. Furthermore, the ratio was calculated as a relative value such that the IL-6 / G3PDH value of the sample of the unstimulated material was defined as "1 (one)". The results are shown in Table 1.

Table 1

| The expression level of the IL-6 gene after contact with the surface of the hollow fiber material (Relative value ±SD, when one not yet in contact with the material is defined as 1) | | | | |
|---|---|---|---|---|
| Sample Name | Example 3 | | Example 4 | Comparative Example 1 |
| | (IL-6/β-actin) | (IL-6/β-actin) | (IL-6/G3PDH) | (IL-6/G3PDH) |
| APS | 1.09±0.17 | 1.06±0.17 | 1.00±0.11 | 1.30±1.33 |
| BK | 2.34±0.37 | 2.45±0.40 | 2.26±0.34 | 1.31±0.12 |
| Without hollow fiber material | 1.01±0.15 | 1.00±0.06 | 1.01±0.19 | 1.00±0.21 |

In the real-time PCRmethods in Examples 3 and 4, the similar results are obtained regardless of differences found in their methods of detecting PCR products and house-keeping genes used as internal standards. However, the quantified value of Comparative Example 1 with the PCR method generally used in the art shows a large standard deviation of the measured value. Thus, a difference in the expression levels of the IL-6 gene could not be detected even though the difference was about twice as high as that of the BK-contacting sample and the difference was detectable in the real-time PCR method. Therefore, it was indicated that the method of the present invention can detect subtle activation of leukocytes simply, quantitatively, and highly sensitively, compared with the conventional method.

(Comparative Example 2)

In Biomaterials 19: 821-827 (1998) by Shinya Kato et al., there is reported that the expression level of the heat shock protein gene of the cells brought into contact with the surface of a material can be provided as a biocompatibility marker.

The real-time PCR quantification on the HSP70B gene was carried out using the PCR template of Example 2 in the Light Cycler Quick System 330 (manufactured by Roche Diagnostics Co. , Ltd. , Germany). In addition, as internal standards, the β-actin gene and the G3PDH (GAPDH) gene, which were house keeping genes, were quantified. A hybridization probe method was used as a detection method for β-actin and the Light Cycler™ Fast Start DNA Master Hybridization Probes (manufactured by Roche Diagnostics, Co. , Ltd. , Germany) was used. The SYBR Green I method was used as a detection method of HSP70B and G3PDH (GAPDH) and the Light Cycler™ Fast Start DNA Master SYBR Green I (manufactured by Roche Diagnostics, Co., Ltd., Germany) was used.

The following were used as primers and probes: (SEQ ID NO. 5: 5'-CCAACCGCGAGAAGATGAC-3', SEQ ID NO. 6: 5'-GGAAGGAAGGCTGGAAGAGT-3', SEQ ID NO. 7: 5'-CCTCCCCCATGCCATCCTGCGTC-FITC-3', and SEQ ID NO. 8: 5'-LCRed-GGACCTGGCTGGCCGGGACCTGA-Phosphorylated-3') for β-actin; (SEQ ID NO. 15: 5'-CACTGACAGGAGCACAGGT-3', and SEQ ID NO. 16: 5'-GGACTTCCCGACACTTGTCT-3') for HSP70B; and (SEQ ID NO. 9: 5'-TGAACGGGAAGCTCACTGG-3' and SEQ ID NO. 10: 5'-TCCACCACCCTGTTGCTGTA-3') for G3PDH (GAPDH). Capillaries used for the PCR reaction were Light Cycler™ Capillaries (manufactured by Roche Diagnostics Co., Ltd. , Germany). The concentration of $Mg^{2+}$ in a PCR reaction solution was 2mM in the case of HSP70B, and 3 mM in the other cases. The temperature cycle of the PCR was (95°C for 10 minutes, followed by the 40-cycle repetition

of three-temperature cycle, 95°C for 10 seconds, 56°C for 8 seconds, and 72°C for 13 seconds) for HSP70B, (95°C for 10 minutes, followed by the 40-cycle repetition of three-temperature cycle, 95°C for 15 seconds, 59°C for 15 seconds, and 72°C for 19 seconds) for β-actin, (95°C for 10 minutes, followed by the 40-cycle repetition of four-temperature cycle, 95°C for 10 seconds, 55°C for 5 seconds, 72°C for 13 seconds, and 85°C for 3 seconds) for G3PDH (GAPDH). An analysis on each sample normalized the expression level of the HSP70B gene in each sample by the expression level of the β-actin gene or the expression level of the G3PDH gene and then calculated the ratio of HSP70B / β-actin or HSP70B / G3PDH. Furthermore, each of the ratios was calculated as a relative value such that the HSP70B /β-actin or HSP70B / G3PDH value of the sample of the unstimulated material was defined as "1 (one)". The results are shown in Table 2.

Table 2

| The expression level of the HSP70B gene after contact with the surface of the hollow fiber material (Relative value ±SD, when one not yet in contact with the material is defined as 1) | | |
|---|---|---|
| Sample Name | Comparative Example 2 | |
| | (HSP70B/β-actin) | (HSP70B/G3PDH) |
| APS | 1.21±0.22 | 1.17±0.25 |
| BK | 1.29±0.14 | 1.20±0.10 |
| Without hollow fiber material | 1.01±0.20 | 1.01±0.21 |

Comparing with the case in which the expression level of the IL-6 gene was used as a marker as shown in Table 1, no difference between the APS and BK materials could be found in the case of using the HSP70B gene as a marker as shown in Table 2 even though the real-time PCR method was used.

(Example 5)

All of the devices used for the experiment were pyrogen-free up to the step of cell lysis to avoid endotoxin contamination.

From the elbow vein of the healthy donor, 60 ml of blood was drawn with a disposable syringe and a 19G winged needle. Then, the blood was gently transferred into a 200-ml PP container (polypropylene, 2075, manufactured by Falcon Co., Ltd., U.S.A.) containing 300 µl of a heparin sodium solution (manufactured by Shimizu Pharmaceutical, Co. , Ltd. , Japan) without any delay, and the whole was then mixed gently, followed by being kept at room temperature before the experiment (final heparin concentration: 5 U/ml).

As a hollow fiber material, a regenerated cellulose membrane was prepared as follows. A cupra ammonium cellulose solution with a cellulose concentration of 8% formed as a spinning solution by the known method and a tetrafluoroethane gas as a hollow-forming agent were prepared. The solution and the gas were discharged into the air from double spinnerts at rates of 12.9 ml/min and 3.34 ml/min, respectively, followed by being solidified with a 11% NaOH aqueous solution after allowing the solution and the gas to fall about 55 cm in the air. Subsequently, the solidified product was refined with about 2% sulfate aqueous solution and was washed with water. After that, the product was treated with glycerin, followed by drying. The resulting hollow fiber membrane was of 180 µm in inner diameter and 10 µm in film thickness (hereinafter, referred to as "a cellulose hollow fiber").

A mini-module of 180 mm in length having 200 filaments was formed. Immediately before use, the mini-module was dipped in 10 ml of a physiological saline solution and was then removed from the solution. In addition, a line for the circulation and flow of blood was formed by a tigon tube R-3603 (3.15 " in inner diameter) (manufactured by Norton K.K.). Then, endotoxin was inactivated by washing the line with a physiological saline solution after treating with NaOH. In an incubator (NIB-90, manufactured by Iwaki Co., Ltd., Japan) at 37°C, 5 ml of the heparin-added human fresh blood was circulated in the mini-module to flow the blood at a flow rate of 3 ml/min for 60 seconds using a 15-ml PP tube (polypropylene, 2327-015, manufactured by Iwaki Co., Ltd., Japan), a cassette tube pump SMP-23S (manufactured by Eyela Co. , Ltd. , Japan) , and the tigon tube (material: blood contact surface area ratio = 40.8 $cm^2$/ml). Furthermore, the blood was transferred into a 15-ml PP tube (polypropylene, 2327-015, manufactured by Iwaki Co., Ltd., Japan) and was then incubated at 37°C for 1 hour. One of the comparative examples was performed with the circulation of blood only with the circuit without connection with the mini-module and the other of them was performed with contact with the material without the circulation of blood (untreated).

The blood after contact with the material was gently layered in a 15-ml PS tube (polystyrene propylene, 2321-015, manufactured by Iwaki Co. , Ltd. , Japan) containing 3 ml of a Ficoll-Paque solution (manufactured by Amersham Pharmacia Co. , Ltd. , U.S.A.) so as to form an interface therein, and the whole was then centrifugedby 400 g

for 30 minutes at room temperature, followed by collecting a monocyte layer. Subsequently, 10 ml of a physiological saline solution was added to the layer and the whole was centrifuged by 400 g for 10 minutes at room temperature to obtain a washed monocyte pellet. The cell pellet was transferred into a 1. 5-ml PP tube (polypropylene, Safe Seal Microcentrifuge Tubes, Sorenson BioScience, Inc.), followed by dissolving the cells with 1 ml of Isogen (manufactured by Nippon Gene Co., Ltd., U.S.A.) and extracting the total RNA from the solution as described in the instruction manual. This total RNA (1 μg) was treated with DNase I (Amplification Grade) (manufactured by Gibco BRL Co., Ltd., U.S.A.) as described in the instruction manual, followed by adding oligo dT (manufactured by Gibco BRL Co., Ltd., U.S.A.) therein to prepare cDNA using the Superscript II RT (manufactured by Gibco BRL Co., Ltd., U.S.A.) as described in the instruction manual. The cDNA thus prepared was diluted to a level of 5 ng/μl on the basis of the level of the total RNA being fed. Then, the cDNA was used as a template in a PCR reaction.

The real-time PCR quantification on the IL-6, IL-1β, TNFα, and MCP-1 genes was carried out using the above PCR template in the Light Cycler Quick System 330 (manufactured by Roche Diagnostics Co., Ltd., Germany). In addition, as an internal standard, the β-actin gene, which was one of the house keeping genes, was quantified. The same method as that of Example 4 was used as a detection method for IL-6 and β-actin, and for IL-1β, IL-8, and TNFα, a hybridization probe method was used, and the Light Cycler™ Fast Start DNA Master Hybridization Probes (manufactured by Roche Diagnostics, Co., Ltd., Germany) was used. The SYBR Green I method was used as a detection method for MCP-1 and the Light Cycler™ Fast Start DNA Master SYBR Green I (manufactured by Roche Diagnostics, Co., Ltd., Germany) was used. The following were used as primers and probes: the same primers and probes as those of Example 4 for IL-6 and β-actin; (SEQ ID NO. 17: 5'-CCTGCGTGTTGAAAGATGAT-3', SEQ ID NO. 18: 5'-GGAAGACACAAATTGCATGG-3', SEQ ID NO. 19: 5'-AACAAGCTGGAATTTGAGTCTGCC-FITC-3', and SEQ ID NO. 20: 5'-LCRed-AGTTCCCCAACTGGTACATCAGCA-Phosphorylated-3') for IL-1β; (SEQ ID NO. 21: 5'-TAAACATGACTTCCAAGCTG-3', SEQ ID NO. 22: 5'-CCTCTTCAAAAACTTCTCCA-3', SEQ ID NO. 23: 5'-GGTTTGGAGTATGTCTTTATGCACTGAC-FITC-3', and SEQ ID NO. 24: 5'-LCRed-TCTAAGTTCTTTAGCACTCCTTGGCAA-Phosphorylated-3') for IL-8; and (SEQ ID NO. 25: 5'-CAAGCCTGTAGCCCATGTT-3', SEQ ID NO. 26: 5'-ATGGCAGAGAGGAGGTTGAC-3', SEQ ID NO. 27: 5'-GGGCCTGTACCTCATCTACTCCCA-FITC-3', and SEQ ID NO. 28: 5'-LCRed-GTCCTCTTCAAGGGCCAAGGCT-Phosphorylated-3') for TNFα, and (SEQ ID NO. 29: 5'-AGATGCAATCAATGCCCCAG-3', and SEQ ID NO. 30: 5'-AGTCTTCGGAGTTTGGGTTTG-3') for MCP-1. Capillaries used for the PCR reaction were Light Cycler™ Capillaries (manufacturedby Roche Diagnostics Co., Ltd., Germany) . The concentration of $Mg^{2+}$ in a PCR reaction solution was 2mM in the cases of IL-1β and MCP-1, and 3 mM for the other cases. The temperature cycle of the PCR was the same as that of Example 4 for IL-6 and β-actin, (95°C for 10 minutes, followed by the 40-cycle repetition of three-temperature cycle, 95°C for 15 seconds, 56°C for 10 seconds, and 72°C for 13 seconds) for IL-1β and IL-8, (95°C for 10 minutes, followed by the 40-cycle repetition of three-temperature cycle, 95°C for 15 seconds, 59°C for 15 seconds, and 72°C for 11 seconds) for TNFα, and (95°C for 10 minutes, followedby the 40-cycle repetition of three-temperature cycle, 95°C for 10 seconds, 56°C for 8 seconds, and 72°C for 13 seconds) for MCP-1. An analysis on each sample normalized the expression level of each gene in each sample by the expression level of the β-actin gene and then calculated the ratio of each gene / β-actin. Furthermore, each ratio was calculated as a relative value such that the each gene /β-actin value of the sample of the unstimulated material was defined as "1 (one) ". The results are shown in Table 3.

Table 3

| The expression level of each gene after contact with the surface of the hollow fiber material (Relative value when one not yet in contact with the material is defined as 1) | | | |
|---|---|---|---|
| Gene | Sample | | |
| | Cellulose hollow-fiber module | Only circuit | Untreated |
| IL-1β | 22.6 | 0.8 | 1.0 |
| IL-8 | 5.1 | 1.4 | 1.0 |
| IL-6 | 20.4 | 0.4 | 1.0 |
| TNFα | 5.4 | 2.4 | 1.0 |
| MCP-1 | 39.4 | 3.6 | 1.0 |

In any evaluated gene, comparing with one not yet in contact with the material (untreated) and one with only a circuit, the expression being induced was able to be quantified by bringing the blood into circulative contact with the cellulose hollow-fiber module.

(Example 6)

All of the devices used for the experiment were pyrogen-free up to the step of cell lysis to avoid endotoxin contamination.

From the elbow vein of the healthy donor, 60 ml of blood was drawn with a disposable syringe and a 19G winged needle. Then, the blood was gently transferred into a 200-ml PP container (polypropylene, 2075, manufactured by Falcon Co., Ltd., U.S.A.) containing 300 μl of a heparin sodium solution (manufacturedby Shimizu Pharmaceutical, Co. , Ltd. , Japan) without any delay, and the whole was then mixed gently, followed by being kept at room temperature before the experiment (final heparin concentration: 5 U/ml).

The cellulose hollow fiber described in Example 5 was used as a hollow fiber material.

A mini-module of 110 mm in length having 300 filaments was formed. Immediatelybefore use, themini-modulewas flowed in 5 ml of aphysiological saline solution and was then removed from the solution. From the upper end of the mini-module, 3 ml of the heparin-added human fresh blood was gently flowed and was directly held in the hollow fiber module (material: blood contact surface area ratio = 222 $cm^2$/ml). Immediately after that, the resultant product was incubated in an incubator (NIB-90, manufactured by Iwaki Co., Ltd., Japan) at 37 °C for 30 minutes. The blood being held was collected into a 15-ml PP tube (polypropylene, 2327-015, manufactured by Iwaki Co., Ltd., Japan) by centrifugation, and then the blood was additionally incubated at 37°C for 1 hour. The comparative control was performed with one without contact with the mini-module (untreated).

The total RNA was extracted from the blood after contacting the material, by using the QIAamp RNA Blood Mini Kit (manufactured by Qiagen Ltd.) and following its instruction manual. This total RNA (0.5 μg) was treated with DNase I (Amplification Grade) (manufactured by Gibco BRL Co., Ltd., U.S.A.) as described in the instruction manual, followed by adding oligo dT (manufactured by Gibco BRL Co., Ltd., U.S.A.) therein to prepare cDNA using the Superscript II RT (manufactured by Gibco BRL Co., Ltd. , U.S.A.) as described in the instruction manual. The cDNA thus prepared was diluted to a level of 5 ng/μl on the basis of the level of the total RNA being fed. Then, the cDNA was used as a template in a PCR reaction.

The real-time PCR quantification on the IL-6, IL-1β, IL-8, IL-1α, and MCP-1 genes was carried out using the above PCR template in the Light Cycler Quick System 330 (manufactured by Roche Diagnostics Co., Ltd., Germany). In addition, as an internal standard, the G3PDH(GAPDH) gene, which was one of the house keeping genes, was quantified. The same method as that of Example 4 was used as a detection method for IL-6 and G3PDH (GAPDH) , and for IL-1β, IL-8, and MCP-1, the same method as that of Example 5 was used. The SYBR Green I method was used as a detection method for IL-1α and the Light Cycler™ Fast Start DNA Master SYBR Green I (manufactured by Roche Diagnostics, Co., Ltd., Germany) was used. The following were used as primers and probes: the same primers and probes as those of Example 4 for IL-6 and G3PDH (GAPDH) ; the same primers and probes as those of Example 5 for IL-1β, IL-8, and MCP-1; (SEQ ID NO. 31: 5'-CAAGATGAAGACCAACCAGT-3', and SEQ ID NO. 32: 5'-AAGTCAGTGATAGAGGGTGG-3') for IL-1α. Capillaries used for the PCR reaction were Light Cycler™ Capillaries (manufactured by Roche Diagnostics Co., Ltd., Germany). The concentration of $Mg^{2+}$ in a PCR reaction solution was the same as that of Example 4 for IL-6 and G3PDH (GAPDH) , the same as that of Example 5 for IL-1β, IL-8, and MCP-1, and 3 mM for IL-1α. The temperature cycle of the PCR was the same as that of Example 4 for IL-6 and G3PDH (GAPDH) , the same as that of Example 5 for IL-1β, IL-8, andMCP-1, (95°C for 10 minutes, followed by the 40-cycle repetition of four-temperature cycle, 95°C for 10 seconds, 55°C for 5 seconds, 72 °C for 13 seconds, and 81 °C for 3 seconds) for IL-1α. An analysis on each sample normalized the expression level of each gene in each sample by the expression level of the G3PDH gene and then calculated the ratio of each gene / G3PDH. Furthermore, each ratio was calculated as a relative value such that the each gene / G3PDH value of the sample of the unstimulated material was defined as "1 (one)". The results are shown in Table 4.

Table 4

| The expression level of each gene after contact with the surface of the hollow fiber material (Relative value when one not yet in contact with the material is defined as 1) | | |
|---|---|---|
| Gene | Sample | |
| | Cellulose hollow-fiber module | Untreated |
| IL-1β | 4.9 | 1.0 |
| IL-8 | 4.7 | 1.0 |
| IL-6 | 3.1 | 1.0 |
| IL-1α | 6.1 | 1.0 |
| MCP-1 | 8.3 | 1.0 |

In any one of the evaluated genes, comparing with one not yet in contact with the material (untreated) , the expression being induced was able to be quantified by bringing the blood into hold contact with the cellulose hollow-fiber module. In addition, the expression was able to be simply evaluated by extracting an RNA fraction from the whole blood containing all fractions of leukocytes.

(Example 7)

The influence of the endotoxin contamination in the cellulose hollow fiber used in each of Examples 5 and 6 on the gene expression was studied.
All of the devices used for the experiment were pyrogen-free up to the step of cell lysis to avoid endotoxin contamination.
From the elbow vein of the healthy donor, 60 ml of blood was drawn with a disposable syringe and a 19G winged needle. Then, the blood was gently transferred into a 200-ml PP container (polypropylene, 2075, manufactured by Falcon Co., Ltd., U.S.A.) containing 300 µl of a heparin sodium solution (manufactured by Shimizu Pharmaceutical, Co. , Ltd. , Japan) without any delay, and the whole was then mixed gently, followed by being kept at room temperature before the experiment (final heparin concentration: 5 U/ml).
The cellulose hollow fiber described in Example 5 was used as a hollow fiber material. A mini-module of 110 mm in length having 300 filaments (hereinafter, referred to as "a cellulose module") was formed. Then, 3 ml of a solution of 1 µg/ml LPS (*E. coli* Serotype 026: B6 , manufactured by Sigma Co., Ltd., U.S.A.) was gently flowed into the cellulose module and the LPS solution was then directly held in the hollow fiber module. The resultant product was incubated at room temperature for 60 minutes and the solution was then removed, followed by washing with 10 ml of a physiological saline solution to prepare a module to be artificially contaminated with endotoxin (hereinafter, referred to as "an LPS-contaminated module"). Furthermore, 3 ml of a 0.5N NaOH solution was gently flowed into the LPS-contaminated module and was then directly held in the hollow fiber module, followed by leaving the resultant product as it is at 50°C for 2 hours. After that, washing was performed by removing the solution and dipping with 10 ml of a physiological saline solution. After the confirmation of the fact that the pH of the physiological saline solution used for the washing was neutral, the module with inactivated endotoxin (hereinafter, referred to as "an LPS-inactivated module") was prepared. Furthermore, an untreated module only subjected to the NaOH treatment (hereinafter, referred to as "a NAOH-treated module") was prepared.
Immediately before using the several kinds of mini-modules described above, the modules were dipped in 5 ml of a physiological saline solution and then removed therefrom. From the upper end of the mini-module, 3 ml of the heparin-added human fresh blood was gently flowed and directly held in the hollow fiber module (material: blood contact surface area ratio = 222 $cm^2$/ml). Immediately after that, the resultant product was incubated in an incubator (NIB-90, manufactured by Iwaki Co. , Ltd. , Japan) at 37°C for 30 minutes. The blood being held was collected into a 15-ml PP tube (polypropylene, 2327-015, manufactured by Iwaki Co. , Ltd. , Japan) by centrifugation, and then the blood was additionally incubated at 37°C for 1 hour. The comparative control was performed with one out of contact with the mini-module (untreated).
The total RNA was extracted from the blood after contacting the material, by using the QIAamp RNA Blood Mini Kit (manufactured by Qiagen Ltd.) and following its instruction manual. This total RNA (0.5 µg) was treated with DNase I (Amplification Grade) (manufactured by Gibco BRL Co., Ltd., U.S.A.) as described in the instruction manual, followed by adding oligo dT (manufactured by Gibco BRL Co., Ltd., U.S.A.) therein to prepare cDNA using the Superscript II RT (manufactured by Gibco BRL Co., Ltd. , U.S.A.) as described in the instruction manual. The cDNA thus prepared was diluted to a level of 5 ng/µl on the basis of the level of the total RNA being fed. Then, the cDNA was used as a template in a PCR reaction.
The real-time PCR quantification on the IL-6 was carried out in the same manner as in Example 4 using the above PCR template in the Light Cycler Quick System 330 (manufactured by Roche Diagnostics Co., Ltd., Germany). In addition, as an internal standard, the G3PDH (GAPDH) gene that was a house keeping gene was quantified. An analysis thereon normalized the expression level of each gene in each sample by the expression level of the G3PDH gene and then calculated the ratio of each gene / G3PDH. Furthermore, each ratio was calculated as a relative value such that the each gene / G3PDH value of the sample of the unstimulated material was defined as "1 (one)". The results are shown in Table 5.

Table 5

| The expression level of the IL-6 gene after contact with the surface of the hollow fiber material (Relative value when one not yet in contact with the material is defined as 1) | |
|---|---|
| Sample | Expression level of IL-6 gene |
| Cellulose module | 3.1 |
| LPS-contaminated module | 233.2 |
| LPS-inactivated module | 2.8 |
| NaOH-treated module | 3.1 |
| Not yet in contact with hollow fiber material | 1.0 |

It is found that the artificial endotoxin (LPS) contamination exerts a great influence on the expression of the IL-6 gene.

In addition, the "cellulose module", "LPS-inactivated module", and "NAOH-treated module" show similar values. Thus, it is found that the endotoxin level of the cellulose module is insufficient to influence the gene expression and there is no influence of the NaOH treatment on the expression level of the gene while there is a possibility of inactivating the LPS with the NaOH treatment. Furthermore, the expression of the IL-6 gene is induced more than one in a state of being not brought into contact with the material. Thus, it is found that the method of the present invention allows an evaluation on the true biocompatibility of the material itself.

(Example 8)

All of the devices used for the experiment were pyrogen-free up to the step of cell lysis to avoid endotoxin contamination.

From the elbow vein of the healthy donor, 100 ml of blood was drawn with a disposable syringe and a 19G winged needle. Then, the blood was gently transferred into a 200-ml PP container (polypropylene, 2075, manufactured by Falcon Co., Ltd., U.S.A.) containing 1000 "1 of a heparin sodiumsolution (manufactured by Shimizu Pharmaceutical, Co., Ltd., Japan) without any delay, and the whole was then mixed gently, followed by being kept at room temperature before the experiment (final heparin concentration: 5 U/ml).

AM-SD-150U (manufactured by Asahi Medical Co., Ltd., Japan) was used as a hollow fiber material. After the inner and outer surfaces of a hollow fiber had been washed with 500 ml of a physiological saline solution (Otsuka Pharmaceutical Co., Ltd., Japan) in a clean bench, AM-SD-150U (made of a recycled cellulose film, hereinafter referred to as "AM-SD") was aseptically decomposed by a dialyzer, followed by removing out the hollow fiber to provide a sample material under test.

The hollow fiber material was aseptically cut into pieces in a clean bench with an external surface area of 80 $cm^2$ and placed in 50-ml PP tubes (polypropylene, 2070 Falcon Co., Ltd., U.S.A.), followedbywashing three times with 20 ml of a physiological saline solution. Then, 15 ml of the blood was added to each of them and each mixture was then brought into contact with the material in a water bath (Thermo Minder DX-10, manufactured by Taitec Co., Ltd., Japan) at 37°C for 60 minutes, while reverse-mixing occasionally at intervals of 10 minutes (material: blood contact surface area ratio 5.3 $cm^2$/ml). As a comparative control, the same procedure was performed without the addition of the hollow fiber material. The blood after contact with the material was gently layered in each of several 15-ml PS tubes (polypropylene, 2321-015, manufactured by Iwaki Co., Ltd., Japan) containing 3 ml of a Ficoll-Paque solution (manufactured by Amersham Pharmacia Co., Ltd., U.S.A.) so as to form an interface therein, and each mixture then centrifuged by 400 g for 30 minutes at room temperature, followed by collecting a mononuclear cell layer. Subsequently, 10 ml of a physiological saline solution was added to the layer, and the whole was centrifuged by 400 g for 10 minutes at room temperature to obtain a washed mononuclear cell pellet.

The mononuclear cell pellet was transferred into a 1.5-ml PP tube (polypropylene, SafeSeal Microcentrifuge Tubes, Sorenson BioScience, Inc.) and then the cells were dissolved with 1 ml of Trizol (manufactured by Gibco BRL Co., Ltd., U.S.A.), followed by extracting the total RNA from the solution as described in the instruction manual. Each sample of total RNA (20 "g) was labeled with Cy5 or Cy3 (manufactured by Amersham Pharmacia Co., Ltd.) to prepare cDNA labeled with Cy5 or Cy3 using the Atlas™ Glass Fluorescent Labeling Kit (manufactured by Clontech Co. , Ltd. , U.S.A.) as described in the instruction manual thereof (at this time, the marker for the cDNA derived from the cells not yet in contact with the material was different from the marker for the cDNA derived from the cells after coming in contact with the material). The cDNA derived from the cells not yet in contact with the material and the cDNA derived from the cells in contact with a given material, which were labeledwith different labels, were mixed with each other in equal amounts to allow the hybridization thereof overnight at 50°C as described in the instruction manual of the Atlas™ Glass

Human 1. 0 Microarray (manufactured by Clontech Co., Ltd., U.S.A.). Scaning data was obtained using the ScanArray Lite (manufactured by GSI Lumonics Co., Ltd., U.S.A.) and the QuantArray ver. 2.0 (manufactured by GSI Lumonics Co., Ltd., U.S.A.), and the conditions for obtaining the data were determined such that the fluorescence intensities of house keeping genes of the Cy5 corresponded with those of the Cy3 with a laser power of 80 V. Furthermore, for analyzing the data, the data was digitalized using the GeneSpring™ 3.0 (manufactured by Silicon Genetics Co., Ltd., U.S.A.).

In the case where a sample brought into contact with AM-SD was labeled with Cy5 and a sample not yet in contact with the material was labeled with Cy3, the fluorescence intensity ratio Cy5/Cy3 was 2.00 for the uPA-R gene. It was found that, by being brought into contact with the AM-SD, the gene expression of the uPA-R increased 2.00 times. Therefore, the biocompatibility of the material was able to be quantified using the gene expression of the uPA-R as a marker. Further, for confirmation, the fluorescent marker was replaced and the sample kept in contact with the AM-SD was labeled with Cy3. The fluorescence intensity ratio Cy3/Cy5 when the sample not yet in contact with the material was labeled with the Cy5 was 1.82 for the uPA-R gene, so that the same data was able to be obtained. In addition, in the case of the other genes, it was found that the IL-1β gene and the IL-8 gene, which were known to increase their gene expressions by contact with a material, showed 20 or more and 15 or less times increases in their gene expressions, respectively. It was indicated that the expression of the uPA-R gene was induced by bringing the surface into contact with a material without depending on the experimental method. Therefore, the uRA-R gene was found out as a candidate evaluation marker with respect to the cell stimulatory of the material.

(Example 9)

Using the blood from another blood donor different from one in Example 8, an experiment identical to that of Example 8 was carried out. As a result, in the case that a sample brought into contact with AM-SD was labeled with Cy5 and a sample not yet in contact with the material was labeled with Cy3, the fluorescence intensity ratio Cy5/Cy3 was 2.23 for the uPA-R gene. It was indicated that Example 8 was not a phenomenon specific to the blood donor and the expression of the uPA-R gene was induced by being brought into contact with the surface of a material. In addition, the uPA-R gene was found out as a candidate evaluation marker with respect to the cell stimulatory of the material.

(Example 10)

All of the devices used for the experiment were pyrogen-free up to the step of cell lysis to avoid endotoxin contamination. In addition, for eliminating the influence of endotoxin which may be slightly causing contamination in the commercially available hollow fiber, the experiment was conducted with the addition of sulfate polymyxin B which was an LPS affinity antibiotic.

From the elbow vein of the healthy donor, 100 ml of blood was drawn with a disposable syringe and a 19G winged needle. Then, the blood was gently transferred into a 200-ml PP container (polypropylene, 2075, manufactured by Falcon Co., Ltd., U.S.A.) containing 1000 µl of a heparin sodium solution (manufactured by Shimizu Pharmaceutical, Co., Ltd., Japan) without any delay (final heparin concentration: 5 U/ml), and the whole was then mixed gently.

Each of AM-SD-150U (manufactured by Asahi Medical Co., Ltd. , Japan), APS-150S (manufactured by Asahi Medical Co., Ltd., Japan), and BK-1.6P (manufactured by Toray Industries, Inc., Japan) were used as a hollow fiber material. Each of the AM-SD-150U (hereinafter, referred to as "AM-SD"), APS-150S (prepared by blending polysulfone and polyvinyl pyrrolidone, hereinafter referred to as "APS"), and BK-1.6P (prepared by blending isotactic polymethyl methacrylate and syndiotactic polymethyl methacrylate, hereinafter referred to as "BK") was provided as a sample material under test, where the inner and outer surfaces of the hollow fiber were washed with 500 ml of a physiological saline solution (Otsuka Pharmaceutical Co. , Ltd. , Japan) in a clean bench on the day of experiment, followed by aseptically decomposing by a dialyzer and pulling out the hollow fiber. In addition, for the BK, a thin fibrous spacer filament inserted between the hollow fibers was aseptically separated therefrom to allow only the hollow fibers to remain in place to provide samplematerials under test. Those hollow fiber materials were aseptically cut into pieces in a clean bench with an external surface area of 80 $cm^2$ and placed in 50-ml PP tubes (polypropylene, 2070 Falcon Co., Ltd., U.S.A.), followed by being stored in syringe water (manufactured by Otsuka Pharmaceutical Co., Ltd., Japan) added with 1 µg/ml sulfate polymyxin B (manufactured by Sigma Co., Ltd., U.S.A.) at 4°C until the day of experiment.

The above hollow fiber material was washed three times with 30 ml of a physiological saline solution (manufactured by Otsuka Pharmaceutical Co., Ltd., Japan) just before the experiment. A mixture prepared by adding sulfate polymyxin B (manufactured by Sigma Co. , Ltd. , U.S.A.) having a final concentration of 10 µg/ml in the previously collected blood was gradually added in increments of 15 ml to bring into contact with the material in a water bath (Thermo Minder DX-10, manufactured by Taitec Co., Ltd., Japan) at 37°C for 60 minutes, while reverse-mixing occasionally at intervals of 10 minutes (material: blood contact surface area ratio = 5.3 $cm^2$/ml). As a comparative control, the same procedure was performed without the addition of the hollow fiber material. The blood after contact with the

material was divided and gently layered in several 15-ml PS tubes (polystyrene, 2321-015, manufactured by Iwaki Co., Ltd., Japan) each containing 3 ml of a Ficoll-Paque solution (manufactured by Amersham Pharmacia Co., Ltd., U.S. A.) so as to form their respective interfaces therein. After the blood had been centrifuged by 400 g for 30 minutes at room temperature, a mononuclear cell layer was obtained, followed by the addition of 10 ml of a physiological saline solution. Then, the washed mononuclear cell pellet was obtained by centrifuging the mononuclear cell layer at 400 g for 30 minutes at room temperature.

The cell pellet was transferred into a 1. 5-ml PP tube (polypropylene, Safe Seal Microcentrifuge Tubes, Sorenson BioScience, Inc.), followed by dissolving the cells with 1 ml of Trizol (manufactured by Gibco BRL Co., Ltd., U. S.A.) and extracting the total RNA from the solution as described in the instruction manual. The total RNA (1 μg) of each sample was treated with DNase I (Amplification Grade) (manufactured by Gibco BRL Co., Ltd., U.S.A.) as described in the instruction manual, followed by adding oligo dT (manufactured by Gibco BRL Co., Ltd., U.S.A.) therein to prepare cDNA using the Superscript II RT (manufactured by Gibco BRL Co., Ltd., U.S.A.) as described in the instruction manual. The cDNAs thus prepared were diluted to levels of 50 ng/μl, 10 ng/ml, and 2 ng/ml, respectively on the basis of the level of the total RNA being fed. Then, the cDNAs were used as templates in a PCR reaction.

The real-time PCR quantification of the IL-6 gene was carried out using the PCR template in the LightCycler Quick System 330 (manufactured by Roche Diagnostics Co., Ltd., Germany). In addition, as an internal standard, the G3PDH (GAPDH) gene, which was of the house keeping genes, was quantified. An SYBR Green I method was used as a detection method for the uPA-R gene and G3PDH (GAPDH) and the Light Cycler™ Fast Start DNA Master SYBR Green I (manufactured by Roche Diagnostics, Co., Ltd., Germany) was used. The following were used as primers: (SEQ ID NO. 33: 5'-ATATGGTAAGAGGCTGTGC-3' and SEQ ID NO. 34: 5'-CACAGTCTGGCAGTCATTA-3') for uPA-R; and (SEQ ID NO. 35: 5'-TGAACGGGAAGCTCACTGG-3' and SEQ ID NO. 36: 5'-TCCACCACCCTGTTGCTGTA-3') for G3PDH (GAPDH). Capillaries used for the PCR reaction were Light Cycler™ Capillaries (manufactured by Roche Diagnostics Co., Ltd., Germany). The concentration of $Mg^{2+}$ in a PCR reaction solution was 3 mM. The temperature cycle of the PCR was 95°C for 10 minutes, followed by the 40-cycle repetition of four-temperature cycle, 95°C for 0 seconds, 55°C for 5 seconds,72°C for 13 seconds, and 85°C for 1 second) for each of uPA-R and G3PDH (GAPDH). An analysis on each sample normalized the expression level of the uPA-R gene in each sample by the expression level of the G3PDH gene and then calculated the ratio of uPA-R / G3PDH. Furthermore, the ratio was calculated as a relative value such that the uPA-R / G3PDH value of the sample not yet stimulated with a material was defined as "1". The values obtained for the respective dilution ratios of the PCR template were averaged. The results are shown in Table 6.

Table 6

| The expression level of the uPA-Rgene after contact with the surface of the hollow fiber material (Relative value when one not yet in contact with the material is defined as 1) | |
|---|---|
| Sample Name | (uPA-R/G3PDH) |
| AM-SD | 3.25 |
| BK | 1.95 |
| APS | 2.16 |
| Without hollow fiber material | 1.00 |

For eliminating the influence of endotoxin which may be causing slight contamination in the commercially available hollow fiber, the experiment was conducted with the addition of sulfate polymyxin B which was an LPS affinity antibiotic. Even in this case, however, it was found that the expression of the uPA-R gene can be induced by being brought into contact with the surface of a material, representing that the expression can become an evaluation marker for the true biocompatibility of the material itself. In addition, it was also indicated that using the expression of the uPA-R gene as a marker allowed a difference between the materials to be evaluated simply, quantitatively, and highly sensitively. In addition, the uPA-R gene was validated as an evaluation marker with respect to the cell stimulatory of the material.

(Example 11)

The influence of endotoxin contamination on the uPA-R gene expression was studied. All of the devices used for the experiment were pyrogen-free up to the step of cell lysis to avoid the endotoxin contamination.

From the elbow vein of the healthy donor, 60 ml of blood was drawn with a disposable syringe and a 19G winged needle. Then, the blood was gently transferred into a 200-ml PP container (polypropylene, 2075, manufactured by Falcon Co., Ltd., U.S.A.) containing 300 μl of a heparin sodium solution (manufactured by Shimizu Pharmaceutical,

Co., Ltd., Japan) without any delay, and the whole was then mixed gently, followed by being kept at room temperature before the experiment (final heparin concentration: 5 U/ml).

AM-SD-150U (manufactured by Asahi Medical Co., Ltd., Japan) was used as a hollow fiber material. After the inner and outer surfaces of a hollow fiber had been washed with 500 ml of a physiological saline solution (Otsuka Pharmaceutical Co., Ltd., Japan) in a clean bench, AM-SD-150U (hereinafter referred to as "AM-SD") was aseptically decomposed by a dialyzer, followed by removing out the hollow fiber to provide a sample material under test. The hollow fiber material was aseptically cut into pieces in a clean bench with an external surface area of 40 $cm^2$ and placed in 15-ml PP tubes (polyprolylene, 2327-015, manufactured by Iwaki Co., Ltd., Japan), followed by washing three times with 5 ml of a physiological saline solution (hereinafter referred to as "untreated SD").

This untreated SD was dipped in 6 ml of a 1-"g/ml or 100-ng/ml solution of LPS (*E. coli* Serotype 026 : B6, manufactured by Sigma Co., Ltd., U.S.A.) and was incubated at room temperature for 90 minutes, followed by washing twice with 10 ml of a physiological saline solution to prepare hollow fibers artificially contaminated with endotoxin (hereinafter, they will be referred to as "SD-LPS 1-"g/ml hollow fiber" and "SD-LPS 100-ng/ml hollow fiber, respectively). In addition, the untreated SD and the SD-LPS 100-ng/ml hollow fiber were flowed in 10 ml of a physiological saline solution containing 5000 unit/ml sulfate polymyxin B (manufactured by Pfizer Pharmaceuticals, Inc., U.S.A.), which was an LPS affinity antibiotic, and the whole was incubated at room temperature for 90 minutes, followed by washing three times with 10 ml of a physiological saline solution to prepare products (hereinafter, they will be referred to as "SD-polymyxin treated hollow fiber" and "SD-LPS 100-ng polymyxin treated hollow fiber", respectively) in which endotoxin was inactivated as far as possible.

Several kinds of the hollow fibers subjected to various treatments described above were washed with 5 ml of a physiological saline solution, followed by removing the physiological saline solution therefrom, just before use. Then, 5 ml of the heparin-added human fresh blood was added to each of them and each mixture was set to the rotator "ROTATOR RT-5" (manufactured by Taitec Co., Ltd., Japan), followed by stirring and contacting at 2 rpm for 60 minutes in an incubator (NIB-90, manufactured by Iwaki Co., Ltd., Japan) at 37°C (material : blood contact surface area ratio = 8 $cm^2$/ml). Then, only the blood was transferred into a 15-ml PP tube (polypropylene, 2327-015, manufactured by Iwaki Co., Ltd., Japan) and incubated at 37°C for 1 hour. As a comparative control, the same procedure was performed without the addition of the hollow fiber material.

The total RNA was extracted from the blood after contacting the material, by using the QIAamp RNA Blood Mini Kit (manufactured by Qiagen Ltd.) and following its instruction manual. This total RNA (0.5 µg) was treated with DNase I (Amplification Grade) (manufactured by Gibco BRL Co., Ltd., U.S.A.) as described in the instruction manual, followed by adding oligo dT (manufactured by Gibco BRL Co., Ltd., U.S.A.) therein to prepare cDNA using the Superscript II RT (manufactured by Gibco BRL Co., Ltd., U.S.A.) as described in the instruction manual. The cDNA thus prepared was diluted to a level of 5 ng/µl on the basis of the level of the total RNA being fed. Then, the cDNA was used as a template in a PCR reaction (data according to this template is described as "WBC fraction" in Table 7).

Further, the blood after contacting the untreated SD was gently layered in a 15-ml PS tube (polypropylene, 2321-015, manufactured by Iwaki Co., Ltd., Japan) containing 3 ml of a Ficoll-Paque solution (manufactured by Amersham Pharmacia Co., Ltd., U.S.A.) so as to form an interface therein. After centrifugation by 400 g for 30 minutes at room temperature, a mononuclear cell layer was collected, followed by the addition of 10 ml of a physiological saline solution. Then, a washed mononuclear cell pellet was obtained by centrifugation by 400 g for 10 minutes at room temperature. The cell pellet was transferred into a 1.5-ml PP tube (polypropylene, Safe Seal Microcentrifuge Tubes, Sorenson BioScience, Inc.), followed by dissolving the cells with 1 ml of Isogen (manufactured by Nippon Gene Co., Ltd., Japan) and extracting the total RNA from the solution as described in the instruction manual. The total RNA (1 µg) was treated with DNase I (Amplification Grade) (manufactured by Gibco BRL Co., Ltd., U.S.A.) as described in the instruction manual, followed by adding oligo dT (manufactured by Gibco BRL Co., Ltd., U.S.A.) therein to prepare cDNA using the Superscript II RT (manufactured by Gibco BRL Co., Ltd., U.S.A.) as described in the instruction manual. The cDNA thus prepared was diluted to a level of 5 ng/µl on the basis of the level of the total RNA being fed. Then, the cDNA was used as a template in a PCR reaction (data according to this template is described as "PBMC fraction" in Table 7).

The real-time PCR quantification of the uPA-R gene was carried out using the PCR template in the LightCycler Quick System 330 (manufactured by Roche Diagnostics Co., Ltd., Germany) as in the case of Example 10, except that the temperature cycle of PCR in Example 10 was 95°C for 10 minutes, followed by the 40-cycle repetition of four-temperature cycle, 95°C for 0 seconds, 55°C for 5 seconds, 72°C for 13 seconds, and 85°C for 3 seconds. In addition, as an internal standard, the G3PDH (GAPDH) gene, which was one of the house keeping genes, was quantified. An analysis on each sample normalized the expression level of the uPA-R gene in each sample by the expression level of the G3PDH gene and then calculated the ratio of uPA-R / G3PDH. Furthermore, the ratio was calculated as a relative value such that the uPA-R / G3PDH value of the sample not yet stimulated with a material was defined as "1". The results are shown in Table 7.

Table 7

| The expression level of the uPA-Rgene after contact with the surface of the hollow fiber material (Relative value when one not yet in contact with the material is defined as 1) | | |
|---|---|---|
| Sample | Expression level of uPA-R gene | |
| | WBC fraction | PBMC fraction |
| Untreated SD | 7.5 | 7.5 |
| SD-LPS 100-ng/ml hollow fiber | 6.9 | - |
| SD-LPS 1-"g/ml hollow fiber | 12.2 | - |
| SD-LPS 100-ng polymyxin-treated hollow fiber | 7.9 | - |
| SD-polymyxin-treated hollow fiber | 6.5 | - |
| Not yet in contact with hollow fiber material | 1.0 | - |

A little endotoxin contamination had almost no influence on the expression of the uPA-R gene and there was no significant difference between the "untreated SD" and the "SD-LPS 100-ng/ml hollow fiber". Similar values were obtained in the "SD-LPS 100-ng polymyxin treated hollow fiber" and the "SD-polymyxin treated hollow fiber", and in addition the expression of the uPA-R gene raises in a much endotoxin contamination "SD-LPS 1-"g/ml hollow fiber". Thus, it is found that the "untreated SD" has an endotoxin level insufficient to influence the expression of the gene. Furthermore, it is found that the expression of the uPA-R gene can be induced more in the "untreated SD", compared with a state in which the untreated SD does not come in contact with the material. Therefore, it is found that the true biocompatibility of the material itself can be evaluated according to the method of the present invention.

Furthermore, in the "untreated SD", no difference in the expression levels of the uPA-R gene is found between the WBC fraction and the PBMC fraction. Therefore, it was indicated that any method of extracting an RNA fraction (including a method of extracting the RNA fraction from the total blood containing all fractions of the leukocytes and a method of extracting the RNA fraction after separating a mononuclear cell fraction) was also suitable for the present invention. Furthermore, the uPA-R gene was validated as an evaluation marker with respect to the cell stimulatory of the material.

(Example 12)

All of the devices used for the experiment were pyrogen-free up to the step of cell lysis to avoid endotoxin contamination.

From the elbow vein of the healthy donor, 60 ml of blood was drawn with a disposable syringe and a 19G winged needle. Then, the blood was gently transferred into a 200-ml PP container (polypropylene, 2075, manufactured by Falcon Co., Ltd., U.S.A.) containing 300 µl of a heparin sodium solution (manufactured by Shimizu Pharmaceutical, Co., Ltd., Japan) without any delay, and the whole was then mixed gently, followed by being kept at room temperature before the experiment (final heparin concentration: 5 U/ml).

As a hollow fiber material, a regenerated cellulose membrane was prepared as follows. A cupra ammonium cellulose solution with a cellulose concentration of 8% formed as a spinning solution by the known method and a tetrafluoroethane gas as a hollow-forming agent were prepared. The solution and the gas were discharged into the air from double spinnerets at rates of 12.9 ml/min and 3.34 ml/min, respectively, followed by being solidified with an 11% NaOH aqueous solution after allowing the solution and the gas to fall about 55 cm in the air. Subsequently, the solidified product was refined with an about 2% sulfate aqueous solution and washed with water. After that, the product was treated with glycerin, followed by drying. The resulting hollow fiber membrane was of 180 µm in inner diameter and 10 µm in membrane thickness (hereinafter, referred to as "a cellulose hollow fiber").

A mini-module of 110 mm in length having 300 filaments was formed using the cellulose hollow fiber. Immediately before use, the mini-module was flowed in 5 ml of a physiological saline solution and was then removed therefrom. From the upper end of the mini-module, 3 ml of the heparin-added human fresh blood was gently flowed and directly held in the hollow fiber module (material: blood contact surface area ratio = 222 $cm^2$/ml). Immediately after that, the resultant product was incubated in an incubator (NIB-90, manufactured by Iwaki Co., Ltd., Japan) at 37°C for 30 minutes. The blood being held was collected into a 15-ml PP tube (polypropylene, 2327-015, manufactured by Iwaki Co., Ltd., Japan) by centrifugation, and then the blood was additionally incubated at 37°C for 1 hour. The comparative control was performed with one out of contact with the mini-module (untreated).

The total RNA was extracted from the blood after contacting the material, by using the QIAamp RNA Blood Mini Kit (manufactured by Qiagen Ltd.) and following its instruction manual. This total RNA (0. 5 μg) was treated with DNase I (Amplification Grade) (manufactured by Gibco BRL Co., Ltd., U.S.A.) as described in the instruction manual, followed by adding oligo dT (manufactured by Gibco BRL Co., Ltd., U.S.A.) therein to prepare cDNA using the Superscript II RT (manufactured by Gibco BRL Co., Ltd., U.S.A.) as described in the instruction manual. The cDNA thus prepared was diluted to a level of 5 ng/μl on the basis of the level of the total RNA being fed. Then, the cDNA was used as a template in a PCR reaction.

The real-time PCR quantification on the uPA-R gene was carried out in the same manner as in Example 11 using the above PCR template in the Light Cycler Quick System 330 (manufactured by Roche Diagnostics Co., Ltd., Germany). In addition, as an internal standard, the G3PDH (GAPDH) gene that was a house keeping gene was quantified. An analysis thereon normalized the expression level of the uPA-R in each sample by the expression level of the G3PDH gene and then calculated the ratio of uPA-R / G3PDH. Furthermore, the ratio was calculated as a relative value such that the uPA-R / G3PDH value of the sample of the unstimulated material was defined as "1 (one)". As a result, the expression level of the uPA-R gene was 2.4 (calculated as a relative value when one not yet in contact with the material was defined as "1"). Comparing with one not yet in contact with the material (untreated), the expression being induced was able to be quantified by bringing the blood into hold contact with the cellulose hollow-fiber module. In addition, the uPA-R gene was validated as an evaluation marker with respect to the cell stimulatory of the material.

(Example 13)

From the elbow vein of the healthy donor, 20 ml of blood was drawn with a disposable syringe and a 19G winged needle. Then, the blood was gently transferred into a 50-ml PP container (polypropylene, 2070, manufactured by Falcon Co., Ltd., U.S.A.) containing 100 μl of a heparin sodium solution (manufactured by Shimizu Pharmaceutical, Co., Ltd., Japan) without any delay, and the whole was then mixed gently, followed by being kept at room temperature before the experiment (final heparin concentration: 5 U/ml).

AM-SD-150U (manufactured by Asahi Medical Co., Ltd., Japan, hereinafter referred to as "AM-SD") was used as a hollow fiber material. After the inner and outer surfaces of a hollow fiber had been washed with 500 ml of a physiological saline solution (Otsuka Pharmaceutical Co., Ltd., Japan) in a clean bench, the AM-SD was decomposed by a dialyzer, followed by removing out the hollow fiber to provide a sample material under test.

The hollow fiber material was aseptically cut into pieces in a clean bench with an external surface area of 8 $cm^2$ and placed in siliconized 2-mlPPtubes (the surface thereof was treated with silicon) (polypropylene, Safe Seal Microcentrifuge Tubes, Sorenson BioScience Co., Ltd.), followed by washing three times with 1 ml of a physiological saline solution. Then, 1 ml of blood collected in advance was added therein and the mixture was set to the rotator "ROTATOR RT-5" (manufactured by Taitec Co., Ltd., Japan), followed by stirring and contacting at 2 rpm for 20 minutes in an incubator (NIB-90, manufactured by Iwaki Co., Ltd., Japan) at 37°C (material : blood contact surface area ratio = 8 $cm^2$/ml). As a comparative control, the same procedure was performed without the addition of the hollow fiber material. 100 μl of the blood after being brought into contact with the material was sampled in a 1.5-ml PP tube (polypropylene, manufactured by Toref Co., Ltd., U.S.A) and then 10 μl of the antibody to the uPA-R protein (CD87 R-PE-conjugated mouse anti-human monoclonal antibody: manufactured by Pharmingen Co., Ltd., U.S.A.) was added to the sample, followed by letting the mixture stand at room temperature for 30 minutes. A 1-ml aliquot of a solution prepared by diluting the FACS Lysing solution (Becton Dickinson Co., Ltd., U.S.A.) 10 times with water (hereinafter, referred to as a "hemolytic buffer") was added to suspend a cell pellet, followed by hemolyzing the cells in a dark place at room temperature for 20 minutes. A centrifugation at 10,000 rpm was performed for 30 seconds and then a supernatant was removed, followed by the addition of 100 μl of the hemolyzing buffer again and stirring. Subsequently, 1 ml of a buffer in which BSA (Sigma Co., Ltd.) was dissolved at a concentration of 1% (w/v) in a physiological phosphate buffer solution, pH = 7.4 (hereinafter, referred to as a "washing buffer") was added to the solution, and the whole was then centrifuged at 10,000 rpm for 30 seconds. After the removal of a supernatant, the cell pellet was suspended in 300 μl of 1% paraformardehyde (prepared by diluting "CellFIX" (manufactured by Becton Dickinson, U.S.A.) 10 times with distilled water), providing a sample for measuring the fluorescence intensity thereof.

The fluorescence of cells was measured using the FACSCalibur flow cytometer (manufactured by Becton Dickinson, Co., Ltd., U.S.A.). An automatic adjustment of the cells was performed using the CaliBRITE Beads (manufactured by Becton Dickinson, Co., Ltd., U.S.A.) in a Lyse / wash mode of the FACSComp (manufactured by Becton Dickinson Co. , Ltd. , U.S.A.) , software for automatically setting the conditions of the measurement. After that, the measurement was performed using a 488-nm Ar laser as excitation light at a fluorescence wavelength of 575 ± 15 nm. The measurement data was analyzed using CELL Quest (manufactured by Becton Dickinson Co., Ltd., U.S.A.), data acquisition and analysis software, such that a part where the ranges of forward scattering and lateral scattering corresponding to granulocytes or monocytes was gated. In Table 8, the results of calculating a mean value ± standard deviation (n = 3) of the fluorescence intensities of the cells on the part corresponding to the granulocytes or the part

corresponding to the monocytes are shown. In addition, the typical histograms thereof are shown in Figs. 1 and 2, respectively.

Table 8

| Expression of the uPA-R protein molecule of leukocytes after contacting with the surface of the hollow fiber material | | |
|---|---|---|
| Sample name | Average fluorescence intensity ±SD (n=3) | |
| | Granulocyte | Monocyte |
| AM-SD | 149.21±5.68 | 178.26±5.10 |
| Without hollow fiber material | 47.66±2.19 | 88.53±6.35 |

The results of Table 8 and Figs. 1 and 2 show that the fluorescence intensities of leukocytes (granulocytes and monocytes) in contact with the hollow fiber material (AM-SD) increase comparing with that of the blood out of contact with the hollow fiber material (AM-SD), and thus the expression of the uPA-R protein molecule on the surface of those cells is increased. Therefore, the biocompatibility can be simply evaluated not only by variations in expression of the uPA-R gene but also by variations in expression of the uPA-R protein molecule as markers. In addition, it can be defined that the lower a rate of increasing the fluorescence intensity, the better the biocompatibility. Furthermore, the uPA-Rgene and the uPA-R protein were validated as evaluation markers with respect to the cell stimulatory of the material.

(Example 14)

A hollow fiber material was prepared by the following method (hereinafter, referred to as an "F-PEO 5% hollow fiber").

In a 1000-ml three-neck separable flask, 40.63 g of Bisphenol A (manufactured by Tokyo Kasei Kogyo, Co., Ltd., B0494), 71.57 g of 4,4-dichlorodiphenylsulfone (manufactured by Tokyo Kasei Kogyo Co., Ltd., B0810), 70.0 g of potassium carbonate (manufactured by Wako Pure Chemical Industries, Co., Ltd., 162-03495), 70 ml of toluene (manufactured by Wako Pure Chemical Industries, Co., Ltd., 204-01866), and 182 ml of N-methylpyrrolidone (hereinafter, referred to as "NMP," manufactured by Tokyo Kasei Kogyo Co., Ltd., M0418) were introduced, and the air in the flask was then substituted with nitrogen under stirring. A reaction mixture solution was kept at 155°C and toluene was refluxed for 3 hours. During this period, water distilled off in an azeotropic manner was removed from the reaction mixture solution with a Dean and Stark trap. Subsequently, the reaction mixture solution was heated up to 190°C to distill toluene off, and the solution was kept at 190°C for additional 4 hours to prepare polyethersulfone prepolymer. The obtained polymer had a weight average molecular weight of $4.2 \times 10^3$.

In a 1000-ml three-neck separable flask, 70.24 g of polyethylene glycol#1000 (Sanyo Chemical Industries, Co., Ltd., hydroxyl value = 113 mg KOH/g), 50.99 g of a four functional block copolymer (Tetronic 304, BASF Co., Ltd., hydroxyl value = 139 mg KOH/g) derived from one prepared by successively adding propylene oxide and ethylene oxide to ethylene diamine, 215.0 g of potassium carbonate (Wako Pure Chemical Industries, Co., Ltd., 162-03495) , 120 ml of toluene (Wako Pure Chemical Industries, Co., Ltd., 204-01866), and 290 ml of NMP (manufactured by Tokyo Kasei Kogyo Co., Ltd., M0418) were introduced, and the air in the flask was thensubstitutedwithnitrogenunderstirring. Areactionmixturesolution was kept at 155°C and toluene (Wako Pure Chemical Industries, Co., Ltd., 204-01866) was refluxed for 3.5 hours. During this period, water distilled off in an azeotropic manner was removed from the reaction mixture solution with a Dean and Stark trap. Then, the residue was heated up to 175°C to distill toluene off, followed by adding 8.65 g of 4,4'-difluorodiphenylsulfone (Tokyo Kasei Kogyo Co., Ltd., D0537). Furthermore, the mixture was kept at 175°C for 3.5 hours to prepare a branched PEO prepolymer. The obtained polymer had a weight average molecular weight of $8.0 \times 10^3$.

The PEO prepolymer remaining after the molecular weight measurement was used for preparing a copolymer. A polysulfone prepolymer reaction mixture solution (190°C) in the form of having chlorine on both ends was added with the branched PEO prepolymer reaction mixture solution (155°C) and the whole was maintained as it is at 190°C for 8 hours to synthesize a branched PEO-PSf block copolymer.

The reaction mixture solution was dropped into 8000 ml of water under stirring to obtain a fibrous polymer. A filtrated product was placed in 4000 ml of water, and the mixture was adjusted to pH 2 with concentrated hydrochloric acid, followed by filtrating. Then, washing with water was performed until the filtrate would have a pH of 7, a water-swollen branched PEO-PSf block copolymer was obtained. Then, the water-swollen branched PEO-PSf block copolymer was further washed with 6000 ml of hot water (95°C) for 3 hours, followed by filtrating and vacuum drying at 50°C. The resulting polymer had a weight average molecular weight of $7.4 \times 10^4$.

A spinning solution was prepared, which was composed of 18 parts by weight of aromatic polysulfone (Udel

P-1700; Acomo Engineering Polymers Co., Ltd.), 5 parts by weight of a block copolymer of the branched polyethylene oxide obtained above and aromatic polysulfone, 25 parts by weight of polyethylene glycol 600, and 52 parts by weight of N-methyl-2-pyrrolidone. Then, the spinning solution was kept at 50°C was extruded from double slit spinneret using a mixture solution including 90 parts by weight of water and 10 parts by weight of N-methyl-2-pyrrolidone as an inner cylinder solution, and was dipped in water at 75°C placed 600 mm below the openings, followed by taking up at a rate of 50 m/min. A portion between air gaps was formed of a hood and the inside of the hood was filled with water vapor and kept under the conditions of 40°C in temperature and 90% in humidity. After that, the spun product was washed with hot water (90°C) for 90 minutes and was then dried at 70°C to obtain a hollow fiber for evaluation.

All of the devices used for the experiment were pyrogen-free up to the step of cell lysis to avoid endotoxin contamination.

From the elbow vein of the healthy donor, 60 ml of blood was drawn with a disposable syringe and a 19G winged needle. Then, the blood was gently transferred into a 200-ml PP container (polypropylene, 2075, manufactured by Falcon Co., Ltd., U.S.A.) containing 300 µl of a heparin sodium solution (manufactured by Shimizu Pharmaceutical, Co., Ltd., Japan) without any delay, and the whole was then mixed gently, followed by being kept at room temperature before the experiment (final heparin concentration: 5 U/ml). Bloods of three different healthy donors were respectively used.

A mini-module of 100 mm in length having 300 filaments was formed using the F-PEO 5% hollow fiber. Before use, an inner surface and an outer surface of the hollow fiber were dipped in 20 ml of a physiological saline solution and were then removed therefrom. From the upper end of the mini-module, 3 ml of the heparin-added human fresh blood was gently flowed and directly held in the hollow fiber module (material: blood contact surface area ratio = 200 $cm^2$/ml). Immediately after that, the resultant product was incubated in an incubator (NIB-90, manufactured by Iwaki Co., Ltd., Japan) at 37°C for 30 minutes. The blood being held was collected into a 15-ml PP tube (polypropylene, 2327-015, manufactured by Iwaki Co. , Ltd., Japan) by centrifugation. The comparative control was performed with one out of contact with the mini-module (untreated).

100 µl of the blood after being brought into contact with the material was sampled in a 1.5-ml PP tube (polypropylene, manufactured by Toref Co., Ltd., U.S.A), and then 10 µl of an antibody to the uPA-R protein (CD87 R-PE-conjugated mouse anti-human monoclonal antibody: manufactured by Pharmingen Co., Ltd., U.S.A.) was added to the blood, followed by letting the mixture stand at room temperature for 30 minutes. A 1-ml aliquot of a solution prepared by diluting the FACS Lysing solution (Becton Dickinson Co., Ltd., U.S.A.) 10 times with water (hereinafter, referred to as a "hemolytic buffer") was added to suspend a cell pellet, followed by hemolyzing the cells in a dark place at room temperature for 30 minutes. A centrifugation at 10,000 rpm was performed for 30 seconds and then a supernatant was removed. Subsequently, 1 ml of a buffer in which BSA (Sigma Co., Ltd.) was dissolved at a concentration of 1% (w/v) in a physiological phosphate buffer, pH = 7.4 (hereinafter, referred to as a "washing buffer") was added to the residue, and the whole was then centrifuged at 10,000 rpm for 30 seconds. After the removal of a supernatant, the cell pellet was suspended in 300 "1 of 1% paraformardehyde (prepared by diluting "CellFIX" (manufactured by Becton Dickinson, Co., Ltd., U.S.A.) 10 times with distilled water), thereby providing a sample for measuring the fluorescence intensity thereof.

The fluorescence of cells was measured using the FACS Calibur flow cytometer (manufactured by Becton Dickinson, Co., Ltd., U.S.A.). An automatic adjustment thereof was performed using the CaliBRITE Beads (manufactured by Becton Dickinson, Co., Ltd., U.S.A.) in a Lyse / wash mode of the FACS Comp (manufactured by Becton Dickinson Co., Ltd., U.S.A.), software for automatically setting the conditions of the measurement. After that, the measurement was performed using a 488-nm Ar laser as excitation light at a fluorescence wavelength of 575 ± 15 nm. The measurement data was analyzed using the CELL Quest (manufactured by Becton Dickinson Co., Ltd., U.S.A.), data acquisition and analysis software, such that a portion where the ranges of forward scattering and lateral scatteringcorrespondingtomonocyteswasgated. Bloodsofthreedifferent healthy donors were respectively used to calculate a mean value ± standard deviation (n = 3) of the fluorescence intensities of the cells on the portion corresponding to the monocytes. Further, Table 9 shows magnification levels with respect to the fluorescence intensity of the untreated sample when a similar operation is performed without contacting the material.

(Comparative Example 3)

APS-150E (manufactured by Asahi Medical Co., Ltd., Japan) was treated as described below to be used as a hollow fiber material. After the inner and outer surfaces of the hollow fiber had been washed with 500 ml of injection water (Otsuka Pharmaceutical Co., Ltd., Japan) in a clean bench, the APS-150E (prepared by blending polysulfone and polyvinyl pyrrolidone, hereinafter referred to as "APS-E") was aseptically decomposed by a dialyzer and then the hollow fiber was pulled out thereof and dried with air in the clean bench.

The measurement was performed using the APS-E described above in the same way as that of Example 14 and the results are shown in Table 9. In this case, the inner diameter of the APS-E hollow fiber was 200 µm, so that

the material : blood contact surface area was 200 $cm^2$/ml.

Table 9

| Comparison between the expression of uPA-R protein molecule of monocytes after contact with the surface of the hollow fiber material and that of untreated monocytes | | |
|---|---|---|
| Sample Name | Average fluorescence intensity ±SD (n=3) | Expression strength to untreated |
| F-PEO 5% (Example 14) | 64.41± 5.00 | 1.72 |
| APS-E (Comparative Example 3) | 71.04±11.53 | 1.89 |
| Untreated (Not yet in contact with material) | 37.49± 6.16 | - |

The results in Table 9 show that the F-PEO 5% hollow fiber material described herein is prevented from increasing the expression of uPA-R protein molecule on the surface of monocytes to the surface of the cells after bringing the monocytes into contact with the material, compared with the conventional materials.

When the measurement was conducted under the conditions defined in Claim 14, it can be judged that the material, where the expression level of urokinase plasminogen activator receptor protein on the cell surface was less than 1.8 times as high as that of the human peripheral blood subjected to the same operation without coming in contact with the material, has more excellent biocompatibility than the conventional one.

(Comparative Example 4)

APS-150S (manufactured by Asahi Medical Co., Ltd., Japan) was treated as described below to be used as a hollow fiber material. After the inner and outer surfaces of the hollow fiber had been washed with 500 ml of injection water (Otsuka Pharmaceutical Co., Ltd., Japan) in a clean bench, the APS-150S (prepared by blending polysulfone and polyvinyl pyrrolidone, hereinafter referred to as "APS") was aseptically decomposed by a dialyzer and then the hollow fiber is pulled out thereof and dried in the clean bench.

In addition, the "cellulose hollow fiber" described in Example 5 (it was found to be free of endotoxin contamination as described in Example 7) was also used.

All of the devices used for the experiment were pyrogen-free up to the step of cell lysis to avoid endotoxin contamination.

From the elbow vein of the healthy donor, 60 ml of blood was drawn with a disposable syringe and a 19G winged needle. Then, the blood was gently transferred into a 200-ml PP container (polypropylene, 2075, manufactured by Falcon Co., Ltd., U.S.A.) containing 300 µl of a heparin sodium solution (manufactured by Shimizu Pharmaceutical, Co., Ltd., Japan) without any delay, and the whole was then mixed gently, followed by being kept at room temperature before the experiment (final heparin concentration: 5 U/ml). Blood of one healthy donor was used.

A mini-module of 100 mm in length having 300 filaments was formed usingthe above APS and cellulose hollow fiber. Before use, an inner surface and an outer surface of the hollow fiber were dipped in 20 ml of a physiological saline solution and were then removed therefrom. From the upper end of the mini-module, 3 ml of the heparin-added human fresh blood was gently flowed and directly held in the hollow fiber module. In this case, the inner diameter of the ASP hollow fiber was 200 µm, so that the material : blood contact surface area was 200 $cm^2$/ml. On the other hand, the inner diameter of the cellulose hollow fiber was 180 µm, so that the material : blood contact surface area was 222 $cm^2$/ml.

The results of the measurements performed in the same manner as in Example 14 and Comparative Example 3, except of measuring with n = 3 sampled from the blood of a healthy donor, are shown in Table 10.

Table 10

| Comparison between the expression of uPA-Rproteinmolecule of monocytes after contact with the surface of the hollow fiber material and that of untreated monocytes | | |
|---|---|---|
| Sample Name | Average fluorescence intensity ±SD (n=3) | Expression strength to untreated |
| APS | 135.95±22.12 | 2.24 |
| Cellulose hollow-fiber | 163.69±5.09 | 2.70 |
| Untreated (Not yet in contact with material) | 60.60±2.71 | - |

The results in Table 10 show the material typically used for artificial kidney or the like causes an increase in expression of the uPA-R protein molecule on the surface of monocytes to the surface of the cells after bringing the monocytes into contact with the material.

Industrial Applicability

Using the method of evaluating biocompatibility according to the present invention, it is possible to use cells which can be obtained massively and to measure the activity of blood cells in contact with the surface of the material under completely in vitro conditions without using an extracorporeal circulation circuit or the like. More specifically, the true biocompatibility of the material can be evaluated by, quantifying a change in expression of the gene of cytokine, chemokine, or the like of leukocytes in contact with the surface of the material with a high sensitivity in a simple manner and preventing endotoxin contamination. A novel method of evaluating biocompatibility can be provided by use of a change in expression of a urokinase plasminogen activator receptor gene or a urokinase plasminogen activator receptor protein molecule as a marker, where the activity of nucleated cells in contact with the surface of the material, which is difficult to be detected by the conventional method, can be detectable. Consequently, there is provided a marker effective to the development of a material for treating a body fluid, which does not involve any harmful biological reaction owing to the biocompatibility ofthematerial. In addition, the present invention can be also an effective marker for the development of a material that requires an initiation of blood cell activation. Furthermore, thematerial for treating a body fluid according to the present invention has good biocompatibility in that the conventional method cannot detect the above biocompatibility.

SEQUENCE LISTING

<110> Asahi Kasei Corporation

ASAHI MEDICAL CO., LTD.

<120> Method of Evaluating Biocompatibility

<130> ASAHI-27

<150> JP 2001-236521

JP 2001-236522

<150> 2001-08-03

2001-08-03

<160> 36

<210> 1

<211> 20

<212> DNA

<213> Artificial Sequence

<220>

<223> Sequence from the 245th "A" to the 264th "C" counted from "A" in the start codon of cDNA of human IL-6 gene (GenBank ACCESSION No. M14584).

<400> 1

aagaggcact ggcagaaaac 20

<210> 2

<211> 20

<212> DNA

<213> Artificial Sequence

<220>

<223> Sequence of complementary strand from the 485th "A" to the 504th "C" counted from "A" in the start codon of cDNA of human IL-6 gene (GenBank ACCESSION No. M14584).

<400> 2

gtcaggggtg gttattgcat 20

<210> 3

<211> 28

<212> DNA

<213> Artificial Sequence

<220>

<223> Sequence (3'-end labeled with FITC) from the 393rd "C" to the 420th "C" counted from "A" in the start codon of cDNA of human IL-6 gene (GenBank ACCESSION No. M14584).

<400> 3

cagatttgag agtagtgagg aacaagcc 28

<210> 4

<211> 28

<212> DNA

<213> Artificial Sequence

<220>

<223> Sequence (5'-end labeled with LCRed640 and 3'-end added with a phosphate group) from the 422nd "G" to the 449th "T" counted from "A" in the start codon of cDNA of human IL-6 gene (GenBank ACCESSION No. M14584).

<400> 4

gagctgtcca gatgagtaca aaagtcct 28

<210> 5

<211> 19

<212> DNA

<213> Artificial Sequence

<220>

<223> Sequence from the 341st "C" to the 359th "C" counted from "A" in the start codon of cDNA of human β-actin gene (GenBank ACCESSION No. X00351).

<400> 5

ccaaccgcga gaagatgac 19

<210> 6

<211> 20

<212> DNA

<213> Artificial Sequence

<220>

<223> Sequence of complementary strand from the 780th "A" to the 799th "C" counted from "A" in the start codon of cDNA of human β-actin gene

(GenBank ACCESSION No. X00351).

<400> 6

ggaaggaagg ctggaagagt 20

<210> 7

<211> 23

<212> DNA

<213> Artificial Sequence

<220>

<223> Sequence (3'-end labeled with FITC) from the 510th "C" to the 532nd "C" counted from "A" in the start codon of cDNA of human β-actin gene (GenBank ACCESSION No. X00351)

<400> 7

cctcccccat gccatcctgc gtc 23

<210> 8

<211> 23

<212> DNA

<213> Artificial Sequence

<220>

<223> Sequence (5'-end labeled with LCRed640 and 3'-end added with a phosphate group) from the 534th "G" to the 556th "A" counted from "A" in the start codon of cDNA of human β-actin gene (GenBank ACCESSION No. X00351).

<400> 8

ggacctggct ggccgggacc tga 23

<210> 9

<211> 19

<212> DNA

<213> Artificial Sequence

<220>

<223> Sequence from the 671st "T" to the 689th "G" counted from "A" in the start codon of cDNA of human G3PDH (GAPDH) gene (GenBank ACCESSION No. M33197).

<400> 9

tgaacgggaa gctcactgg 19

<210> 10

<211> 20

<212> DNA

<213> Artificial Sequence

<220>

<223> Sequence of complementary strand from the 958th "T" to the 977th "A" counted from "A" in the start codon of cDNA of human G3PDH (GAPDH) gene (GenBank ACCESSION No. M33197).

<400> 10

tccaccaccc tgttgctgta 20

<210> 11

<211> 24

<212> DNA

<213> Artificial Sequence

<220>

<223> Sequence from the 1st "A" to the 24th "C" counted from "A" in the start codon of cDNA of human IL-6 gene (GenBank ACCESSION No. M14584).

<400> 11

atgaactcct tctccacaag cgcc 24

<210> 12

<211> 21

<212> DNA

<213> Artificial Sequence

<220>

<223> Sequence of complementary strand from the 608th "A" to the 628th "C" counted from "A" in the start codon of cDNA of human IL-6 gene (GenBank ACCESSION No. M14584).

<400> 12

gaagagccct caggctggac t 21

<210> 13

<211> 26

<212> DNA

<213> Artificial Sequence

<220>

<223> Sequence from the 11th "T" to the 36th "T" counted from "A" in the start codon of cDNA of human G3PDH (GAPDH) gene (GenBank ACCESSION No. M33197).

<400> 13

tgaaggtcgg agtcaacgga tttggt 26

<210> 14

<211> 24

<212> DNA

<213> Artificial Sequence

<220>

<223> Sequence of complementary strand from the 970th "G" to the 993rd "G" counted from "A" in the start codon of cDNA of human G3PDH (GAPDH) gene (GenBank ACCESSION No. M33197).

<400> 14

catgtgggcc atgaggtcca ccac 24

<210> 15

<211> 19

<212> DNA

<213> Artificial Sequence

<220>

<223> Sequence from the 1476th "C" to the 1494th "T" counted from "A" in the start codon of cDNA of human HSP70B gene (GenBank ACCESSION No. X51757).

<400> 15

cactgacagg agcacaggt 19

<210> 16

<211> 20

<212> DNA

<213> Artificial Sequence

<220>

<223> Sequence of complementary strand from the 1719th "A" to the 1738th "C" counted from "A" in the start codon of cDNA of human HSP70B gene (GenBank ACCESSION No. X51757).

<400> 16

ggacttcccg acacttgtct 20

<210> 17

<211> 20

<212> DNA

<213> Artificial Sequence

<220>

<223> Sequence from the 557th "C" to the 576th "T" counted from "A" in

the start codon of cDNA of human IL-1β gene (GenBank ACCESSION No. X02532).

<400> 17

cctgcgtgtt gaaagatgat 20

<210> 18

<211> 20

<212> DNA

<213> Artificial Sequence

<220>

<223> Sequence of complementary strand from the 788th "C" to the 807th "C" counted from "A" in the start codon of cDNA of human IL-1β gene (GenBank ACCESSION No. X02532)

<400> 18

ggaagacaca aattgcatgg 20

<210> 19

<211> 24

<212> DNA

<213> Artificial Sequence

<220>

<223> Sequence (3'-end labeled with FITC) from the 670th "A" to the 693rd "C" counted from "A" in the start codon of cDNA of human IL-1β gene (GenBank ACCESSION No. X02532).

<400> 19

aacaagctgg aatttgagtc tgcc 24

<210> 20

<211> 24

<212> DNA

<213> Artificial Sequence

<220>

<223> Sequence (5'-end labeled with LCRed640 and 3'-end added with a phosphate group) from the 695th "A" to the 718th "A" counted from "A" in the start codon of cDNA of human IL-1β gene (GenBank ACCESSION No. X02532).

<400> 20

agttccccaa ctggtacatc agca 24

<210> 21

<211> 20

<212> DNA

<213> Artificial Sequence

<220>

<223> Sequence from the 5th "T" before "A" in the start codon, to the 15th "G" counted from "A" in the start codon of cDNA of human IL-8 gene (GenBank ACCESSION No. Y00787).

<400> 21

taaacatgac ttccaagctg 20

<210> 22

<211> 20

<212> DNA

<213> Artificial Sequence

<220>

<223> Sequence of complementary strand from the 266th "T" to the 285th "G" counted from "A" in the start codon of cDNA of human IL-8 gene (GenBank ACCESSION No. Y00787).

<400> 22

cctcttcaaa aacttctcca 20

<210> 23

<211> 28

<212> DNA

<213> Artificial Sequence

<220>

<223> Sequence (3'-end labeled with FITC) from the 101st "G" to the 128th "C" counted from "A" in the start codon of cDNA of human IL-8 gene (GenBank ACCESSION No. Y00787).

<400> 23

ggtttggagt atgtctttat gcactgac 28

<210> 24

<211> 27

<212> DNA

<213> Artificial Sequence

<220>

<223> Sequence (5'-end labeled with LCRed640 and 3'-end added with a phosphate group) from the 73rd "T" to the 99th "A" counted from "A" in the start codon of cDNA of human IL-8 gene (GenBank ACCESSION No. Y00787).

<400> 24

tctaagttct ttagcactcc ttggcaa 27

<210> 25

<211> 19

<212> DNA

<213> Artificial Sequence

<220>

<223> Sequence from the 258th "C" to the 276th "T" counted from "A" in the start codon of cDNA constructed of spliced exons from the sequence of human TNF-α gene (GenBank ACCESSION No. X02910).

<400> 25

caagcctgta gcccatgtt 19

<210> 26

<211> 20

<212> DNA

<213> Artificial Sequence

<220>

<223> Sequence of complementary strand from the 499th "G" to the 518th "T" counted from "A" in the start codon of cDNA constructed of spliced exons from the sequence of human TNF-α gene (GenBank ACCESSION No. X02910).

<400> 26

atggcagaga ggaggttgac 20

<210> 27

<211> 24

<212> DNA

<213> Artificial Sequence

<220>

<223> Sequence (3′-end labeled with FITC) from the 387th "G" to the 410th "A" counted from "A" in the start codon of cDNA constructed of spliced exons from the sequence of human TNF-α gene (GenBank ACCESSION No. X02910).

<400> 27

gggcctgtac ctcatctact ccca 24

<210> 28

<211> 22

<212> DNA

<213> Artificial Sequence

<220>

<223> Sequence (5'-end labeled with LCRed640 and 3'-end added with a phosphate group) from the 412th "G" to the 433rd "T" counted from "A" in the start codon of cDNA constructed of spliced exons from the sequence of human TNF-α gene (GenBank ACCESSION No. X02910).

<400> 28

gtcctcttca agggccaagg ct 22

<210> 29

<211> 20

<212> DNA

<213> Artificial Sequence

<220>

<223> Sequence from the 75th "A" to the 94th "G" counted from "A" in the start codon of cDNA constructed of spliced exons from the sequence of human MCP-1 gene (GenBank ACCESSION No. M37719).

<400> 29

agatgcaatc aatgccccag 20

<210> 30

<211> 21

<212> DNA

<213> Artificial Sequence

<220>

<223> Sequence of complementary strand from the 277th "C" to the 297th

"T" counted from "A" in the start codon of cDNA constructed of spliced exons from the sequence of human MCP-1 gene (GenBank ACCESSION No. M37719).

<400> 30

agtcttcgga gtttgggtttg 21

<210> 31

<211> 20

<212> DNA

<213> Artificial Sequence

<220>

<223> Sequence from the 586th "C" to the 605th "T" counted from "A" in the start codon of cDNA of human IL-1α gene (GenBank ACCESSION No. X02851).

<400> 31

caagatgaag accaaccagt 20

<210> 32

<211> 20

<212> DNA

<213> Artificial Sequence

<220>

<223> Sequence of complementary strand from the 772nd "C" to the 791st "T" counted from "A" in the start codon of cDNA of human IL-1α gene (GenBank ACCESSION No. X02851).

<400> 32

aagtcagtga tagagggtgg 20

<210> 33

<211> 19

<212> DNA

<213> Artificial Sequence

<220>

<223> Sequence from the 773rd "A" to the 791st "C" counted from "A" in the start codon of cDNA of human uPA-R gene (GenBank ACCESSION No. A18757).

<400> 33

atatggtaag aggctgtgc 19

<210> 34

<211> 19

<212> DNA

<213> Artificial Sequence

<220>

<223> Sequence of complementary strand from the 965th "T" to the 983rd "G" counted from "A" in the start codon of cDNA of human uPA-R gene (GenBank ACCESSION No. A18757).

<400> 34

cacagtctgg cagtcatta 19

<210> 35

<211> 19

<212> DNA

<213> Artificial Sequence

<220>

<223> Sequence from the 671st "T" to the 689th "G" counted from "A" in the start codon of cDNA of human G3PDH (GAPDH) gene (GenBank ACCESSION No. M33197).

<400> 35

tgaacgggaa gctcactgg 19

<210> 36

<211> 20

<212> DNA

<213> Artificial Sequence

<220>

<223> Sequence of complementary strand from the 958th "T" to the 977th "A" counted from "A" in the start codon of cDNA of human G3PDH (GAPDH) gene (GenBank ACCESSION No. M33197).

<400> 36

tccaccaccc tgttgctgta 20

## Claims

1. A method of evaluating biocompatibility of a material, **characterized by** comprising: bringing a surface of the material and an aqueous solution containing a leukocyte into contact with each other *in vitro* for a definite period of time; recovering the aqueous solution containing the leukocyte; extracting an RNA fraction from an inside of the leukocyte; preparing cDNA from the extracted solution using a reverse transcriptase; quantifying an expression level of a specific gene using a real-time PCR method or a DNA array method; and comparing the expression level with an expression level of a specific gene of a leukocyte, which is similarly treated without being brought into

contact with the material.

2. A method of evaluating biocompatibility, **characterized by** comprising: bringing a surface of a material and a nucleated cell-containing solution derived from an animal including a human being into contact with each other for a definite period of time; recovering the solution containing the cells; extracting an RNA fraction from an inside of the cells; preparing cDNA from the extracted solution using a reverse transcriptase; quantifying an expression level of a urokinase plasminogen activator receptor gene; and comparing the expression level with an expression level of a urokinase plasminogen activator receptor gene from nuclear cells derived from an animal including a human being, which are similarly treated without being brought into contact with the material.

3. A method of evaluating biocompatibility according to claim 2, wherein the expression level of the urokinase plasminogen activator receptor gene is quantified using a real-time PCR method or a DNA array method.

4. A method of evaluating biocompatibility, **characterized by** comprising: bringing a surface of a material and a nucleated cell-containing solution derived from an animal including a human being into contact with each other for a definite period of time; recovering the solution containing the cells; quantifying an expression level of a urokinase plasminogen activator receptor protein on the surface of the cells or an intracellular level of the urokinase plasminogen activator receptor protein or an extracellular level of a soluble urokinase plasminogen activator receptor protein; and comparing the expression level with an expression level or a level of a urokinase plasminogen activator receptor protein of nuclear cells derived from an animal including a human being, which are similarly treated without being brought into contact with the material.

5. A method of evaluating biocompatibility according to any one of claims 1 to 3, further comprising the steps of: recovering an aqueous solution containing leukocytes or the nucleated cell-containing solution derived from the animal including a human being; and incubating the nuclear cells at 37°C for 10 minutes to 24 hours, followed by extracting an RNA fraction from an inside of the cells.

6. A method of evaluating biocompatibility according to any one of claims 1 to 5, wherein devices and materials to be used in an experiment have an endotoxin level that does not substantially affect an expression level of a specific gene, and the expression level of the urokinase plasminogen activator receptor gene, the expression level of the urokinase plasminogen activator receptor protein on the surface of the cells, the intracellular level of the urokinase plasminogen activator receptor protein, or the extracellular level of the soluble urokinase plasminogen activator receptor protein.

7. A method of evaluating biocompatibility according to any one of claims 1 to 6, wherein the aqueous solution containing the leukocytes to be brought into contact with the surface of the material or the nuclear cell-containing solution derived from the animal including a human being to be brought into contact with the surface of the material is blood of an animal including a human being or an aqueous solution containing leukocytes isolated from the blood of the animal including the human being, and all of the steps are performed *in vitro*.

8. A method of evaluating biocompatibility according to any one of claims 1 to 7, wherein the aqueous solution containing the leukocytes or the nucleated cell-containing solution derived from the animal including a human being is derived from human normal peripheral blood.

9. A method of evaluating biocompatibility according to any one of claims 1 to 8, wherein a time period for bringing the surface of the material into contact with the aqueous solution containing the leukocytes or a time period for bringing the surface of the material into contact with the nucleated cell-containing solution derived from the animal including a human being is less than 2 hours.

10. A method of evaluating biocompatibility according to any one of claims 1 to 9, wherein the material has one of a hollow fiber form, a filament form, a nonwoven fabric form, a filter form, a flat film form, and a particle form.

11. Amethod of evaluatingbiocompatibility according to any one of claims 1 to 10, wherein the specific gene is at least one of cytokine and chemokine genes or the urokinase plasminogen activator receptor gene.

12. A material for treating a body fluid, **characterized in that** an expression level of a specific gene does not have substantially a difference when an evaluation thereof is performed using the method of evaluating biocompatibility according to any one of claims 1 to 11, compared with a gene expression level of cells which are similarly treated

without being brought into contact with the material.

**13.** Amaterial for treating a body fluid, **characterized in that** the material has substantially no difference when an evaluation thereof is performed using the method of evaluating biocompatibility according to any one of claims 2 to 10, compared with a gene expression level of a urokinase plasminogen activator receptor molecule of nuclear cells which are similarly treated without being brought into contact with the material, an expression level of a urokinase plasminogen activator receptor protein on a surface of the cells, an intracellular level of a urokinase plasminogen activator receptor protein, or an extracellular level of a soluble urokinase plasminogen activator receptor protein out of the nuclear cells in the nuclear cell-containing solution.

**14.** A material for treating a body fluid, **characterized in that**, when human peripheral blood is brought into contact with a surface of a material under the conditions of a contact surface area ratio between the blood and the material of 250 $cm^2$/ml to 160 $cm^2$/ml at 37°C for 30 minutes, an expression level of a urokinase plasminogen activator receptor protein on a cell surface is less than 1.80 times as high as an expression level of a urokinase plasminogen activator receptor protein on a cell surface of human peripheral blood which is similarly treated without being brought into contact with the material.

**15.** A material for treating a body fluid, **characterized in that**, when human peripheral blood is brought into contact with the surface of a material under the conditions of a contact surface area ratio between the blood and the material of 250 $cm^2$/ml to 160 $cm^2$/ml at 37°C for 30 minutes, an expression level of a urokinase plasminogen activator receptor protein on the surface of the cells is less than 1.76 times as high as an expression level of a urokinase plasminogen activator receptor protein on the cell surface of human peripheral blood which is similarly treated without being brought into contact with the material.

**16.** A material for treating a body fluid, **characterized in that**, when human peripheral blood is brought into contact with the surface of a material under the conditions of a contact surface area ratio between the blood and the material of 250 $cm^2$/ml to 160 $cm^2$/ml at 37°C for 30 minutes, an expression level of a urokinase plasminogen activator receptor protein on the surface of the cells is 1.72 times or less as high as an expression level of a urokinase plasminogen activator receptor protein on the cell surface of human peripheral blood which is similarly treated without being brought into contact with the material.

**17.** A material for treating a body fluid according to any one of claims 14 to 16, further comprising an operation of making comparison such that the expression level of the urokinase plasminogen activator receptor protein on the surface of the cells is measured on the surface of a leukocyte in the human peripheral blood.

**18.** A material for treating a body fluid according to claims 14 to 16, wherein an operation of making comparison such that the expression level of the urokinase plasminogen activator receptor protein on the surface of the cells is measured on a surface of monocytes in the human peripheral blood is included.

**19.** A material for treating a body fluid according to any one of claims 12 to 18, wherein the material comprises a hydrophobic polymer and a hydrophilic polymer as main components and the content of the hydrophobic polymer is 50% by weight or more of the total.

**20.** A material for treating a body fluid according to claim 19, wherein the hydrophobic polymer is a polysulfone resin.

**21.** A material for treating a body fluid according to claim 19, wherein the hydrophilic polymer includes polyvinyl pyrrolidone (PVP), polyethylene oxide (PEO) , a copolymer of the PVP or PEO and the hydrophilic polymer, or two or more of the hydrophilic polymers.

**22.** A material for treating a body fluid according to any one of claims 12 to 21, wherein the material has a hollow fiber membrane form.

**23.** A material for treating a body fluid according to claim 22, wherein the hollow fiber membrane has an inner diameter of 100 to 300 μm.

**24.** A material for treating a body fluid according to claim 22, wherein the hollow fiber membrane has an inner diameter of 160 to 250 μm.

**25.** A material for treating a body fluid according to claim 22, wherein a permeation amount of pure water is 5ml/ $m^2$·mmHg·hr or more and 1000 ml/$m^2$·mmHg·hr or less.

**26.** A material for treating a body fluid according to claim 22, wherein a sieving coefficient of β2-microglobulin is 0.4 or more and 1 or less.

**27.** A material for treating a body fluid according to any one of claims 12 to 26, wherein the material is used in blood-treating application.

**28.** A material for treating a body fluid according to any one of claims 12 to 27, wherein the material is used in extracorporeal blood-circulation application of blood.

**29.** A material for treating a body fluid according to any one of claims 12 to 28, wherein the material is one to be used in blood-purificating application by extracorporeal circulation of blood.

**30.** A material for treating a body fluid according to any one of claims 12 to 29, wherein the material is used in an application selected from the group consisting of hemodialysis, hemofiltration, and hemodiafiltration.

**31.** A material for treating a body fluid according to any one of claims 12 to 30, wherein the material is subjected to a sterilization process.

**32.** A material for treating a body fluid according to claim 31, wherein the sterilization process is one selected from the group consisting of high pressure steam sterilization, radiation sterilization and gas sterilization.

**33.** A material for treating a body fluid according to claim 32, wherein the sterilization process is carried out in a dry state or in a wet state.

**34.** A material for treating a body fluid according to claim 32 or 33, wherein the radiation used for the sterilization process is an electron radiation or a gamma radiation.

**35.** A material for evaluating biocompatibility, **characterized by** comprising a nucleic acid having 10 or more residues of a sequence identical to or complementary to cDNA or mRNA of a urokinase plasminogen activator receptor gene.

**36.** A material for evaluating biocompatibility, **characterized by** comprising a material having affinity to a protein molecule of a urokinase plasminogen activator receptor.

**37.** A material for evaluating biocompatibility according to claim 36, wherein the material having the affinity to the protein molecule of the urokinase plasminogen activator receptor is an antibody.

**38.** A method of selecting an evaluation marker for cell stimulatory of a material, comprising: bringing a surface of the material and a nuclear cell-containing solution derived from an animal including a human being into contact with each other for a definite period of time; recovering the solution containing the cells; extracting an RNA fraction from an inside of the cells; preparing cDNA from the extracted solution using a transcriptase; quantifying an expression level of a specific gene using a DNA array method; and defining as a candidate the cell showing a difference in expression levels when compared with the expression level of the specific gene of cells which are similarly treated without being brought into contact with the material.

**39.** A method of selecting an evaluation marker for cell stimulatory of a material according to claim 38, further comprising an operation in which the difference in expression levels of the candidate gene is confirmed based on whether or not the material is brought into contact therewith by using a method of quantifying a gene expression, except for the DNA array method.

**40.** A method of selecting an evaluation marker for cell stimulatory of a material according to claim 38 or 39, further comprising an operation in which the difference in expression levels of the candidate gene is confirmed based on whether or not the material is brought into contact therewith by quantifying a protein encoded by the candidate gene as a protein level in the inside or outside of the cells or on the surface of the cells.

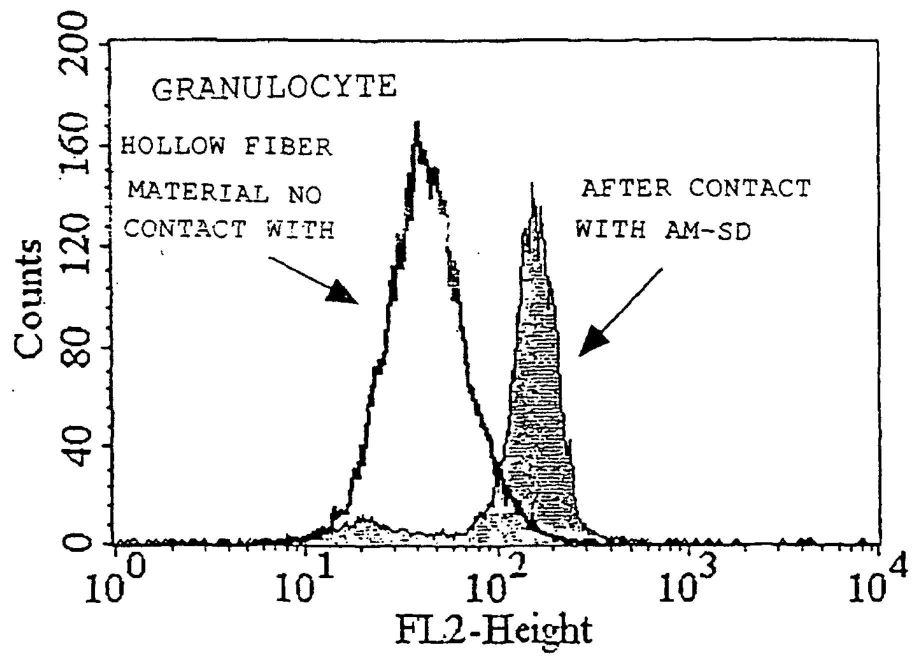
GRANULOCYTE
HOLLOW FIBER
MATERIAL NO
CONTACT WITH
AFTER CONTACT
WITH AM-SD
Counts
0
40
80
120
160
200
$10^0$
$10^1$
$10^2$
$10^3$
$10^4$
FL2-Height

FIG. 1

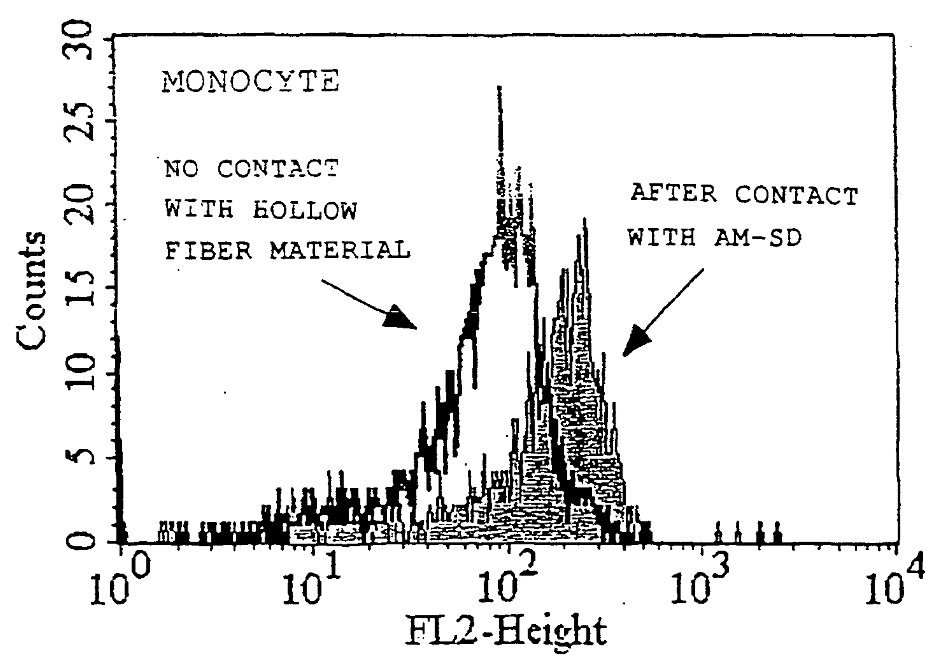
MONOCYTE
NO CONTACT
WITH HOLLOW
FIBER MATERIAL
AFTER CONTACT
WITH AM-SD
Counts
0
5
10
15
20
25
30
$10^0$
$10^1$
$10^2$
$10^3$
$10^4$
FL2-Height

FIG. 2

## INTERNATIONAL SEARCH REPORT

International application No.
PCT/JP02/07896

**A. CLASSIFICATION OF SUBJECT MATTER**

Int.Cl[7] C12Q1/68, 1/02, C12N15/09, G01N33/53, 33/566, A61M1/18

According to International Patent Classification (IPC) or to both national classification and IPC

**B. FIELDS SEARCHED**

Minimum documentation searched (classification system followed by classification symbols)

Int.Cl[7] C12Q1/00-1/70, C12N15/00-15/90, G01N33/53-33/577, A61M1/00-1/38

Documentation searched other than minimum documentation to the extent that such documents are included in the fields searched

Electronic data base consulted during the international search (name of data base and, where practicable, search terms used)

BIOSIS/WPIDS/MEDLINE(STN)

**C. DOCUMENTS CONSIDERED TO BE RELEVANT**

| Category* | Citation of document, with indication, where appropriate, of the relevant passages | Relevant to claim No. |
|---|---|---|
| Y<br>A | WEBER N. et al., Gene monitoring of surface-activated monocytes in circulating whole blood using duplex RT-PCR J.Biomed.Mater.Res., 2001 Jul., 56(1), pages 1 to 8 | 1,5-11,38-40<br>2-4,12-37 |
| Y<br>A | XYNOS I.D. et al., Gene-expression profiling of human osteoblasts following treatment with the ionic products of Bioglass 45S5 dissolution. J.Biomed.Mater.Res., 2001 May, 55(2), p.151-7 | 1,5-11,38-40<br>2-4,12-37 |
| Y<br>A | WO 00/26404 A (Takara Shuzo Co.), 11 May, 2000 (11.05.00), & EP 1126035 A1 | 1,5-11,38-40<br>2-4,12-37 |
| Y<br>A | WO 00/12760 A1 (Incyte Pharmaceuticals Inc.), 09 March, 2000 (09.03.00), & JP 2002-523112 A | 1,5-11,38-40<br>2-4,12-37 |

☒ Further documents are listed in the continuation of Box C.  ☐ See patent family annex.

* Special categories of cited documents:
- "A" document defining the general state of the art which is not considered to be of particular relevance
- "E" earlier document but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| 05 November, 2002 (05.11.02) | 26 November, 2002 (26.11.02) |
| Name and mailing address of the ISA/<br>Japanese Patent Office | Authorized officer |
| Facsimile No. | Telephone No. |

Form PCT/ISA/210 (second sheet) (July 1998)

## INTERNATIONAL SEARCH REPORT

International application No.
PCT/JP02/07896

C (Continuation). DOCUMENTS CONSIDERED TO BE RELEVANT

| Category* | Citation of document, with indication, where appropriate, of the relevant passages | Relevant to claim No. |
|---|---|---|
| Y<br>A | Supervised by Toshiharu MAKI, "Saibo Kogaku Bessatsu Tips Series 'Kaitei PCR Tips'", 2nd edition, Shujunsha, 10 December, 1999 (10.12.99), pages 94 to 99 | 1,5-11,38-40<br>2-4,12-37 |
| X<br>A | WO 92/07083 A1 (Cancerforskiningsfondet AF 1989), 30 April, 1992 (30.04.92),<br>& EP 574391 A1 & JP 7-500486 A<br>& US 5519120 A | 35-37<br>1-34,38-40 |
| A | WO 00/11058 A1 (Circe Biomedical Inc.), 02 March, 2000 (02.03.00),<br>& US 6172180 B1 & EP 1141059 A1<br>& JP 2002-523537 A | 1-40 |
| A | KATO S. et al., Study of cellular responses to polymeric biomaterials using the differential display method. J.Biomater.Sci.Polym.Ed., 2000, 11(4), p.333-40 | 1-40 |
| A | CENNI E. et al., Cytokine expression in vitro by cultured human endothelial cells in contact with polyethylene terephthalate coated with pyrolytic carbon and collagen. J.Biomed.Mater.Res., 15 June, 2000 (15.06.00), 50(4), p.483-9 | 1-40 |
| A | JP 2000-254222 A (Terumo Corp.), 19 September, 2000 (19.09.00), (Family: none) | 1-40 |
| P,A | JP 2002-224545 A (Asahi Kasei Corp.), 05 February, 2001 (05.02.01), (Family: none) | 1-40 |

Form PCT/ISA/210 (continuation of second sheet) (July 1998)

INTERNATIONAL SEARCH REPORT

International application No.
PCT/JP02/07896

Claims 12 to 34 relate to materials for treating body fluids which are specified by using the results of the evaluation methods as set forth in claims 1 to 11. However nothing but only one material for treating body fluids as set forth in claim 14 is supported by the description in the meaning as defined in the Article 6 of the PCT and disclosed therein in the meaning as defined in the Article 5 of the PCT.

Even though the common technical knowledge at the point of the application is taken into consideration, the scope of materials for treating body fluids having been specified by using the results of the evaluation methods as set forth in claims 1 to 11 cannot be specified. Thus, claims 12 to 34 also fail to fulfill the requirement of clearness under the Article 6 of the PCT.

Such being the case, this search was made on the material for treating a body fluid of Example 14 which is specifically disclosed in the description.

Form PCT/ISA/210 (extra sheet) (July 1998)